(19) Europäisches Patentamt
European Patent Office
Office européen des brevets

(11) EP 3 519 578 B1

(12) **EUROPEAN PATENT SPECIFICATION**

(45) Date of publication and mention of the grant of the patent:
**22.12.2021 Bulletin 2021/51**

(21) Application number: **17857727.6**

(22) Date of filing: **02.10.2017**

(51) Int Cl.:
***A61K 9/51*** (2006.01) ***C12N 15/88*** (2006.01)
***C07F 9/10*** (2006.01) ***C07J 41/00*** (2006.01)
***C12N 15/11*** (2006.01)

(86) International application number:
**PCT/CA2017/051167**

(87) International publication number:
**WO 2018/064755 (12.04.2018 Gazette 2018/15)**

(54) **COMPOSITIONS FOR TRANSFECTING RESISTANT CELL TYPES**

ZUSAMMENSETZUNGEN ZUR TRANSFEKTION VON RESISTENTEN ZELLTYPEN

COMPOSITIONS POUR LA TRANSFECTION DE TYPES DE CELLULES RÉSISTANTES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **03.10.2016 US 201662403640 P**

(43) Date of publication of application:
**07.08.2019 Bulletin 2019/32**

(73) Proprietor: **Precision Nanosystems Inc**
**Vancouver, British Columbia V6P 6T7 (CA)**

(72) Inventors:
- **THOMAS, Anitha**
**New Westminster**
**British Columbia V3M 0B2 (CA)**
- **DE SOUZA, Rebecca Anne Grace**
**Vancouver**
**British Columbia V6Z 3G2 (CA)**
- **OUELLET, Eric**
**New Westminster**
**British Columbia V3L 5S2 (CA)**
- **THARMARAJAH, Grace Tharmini**
**Toronto**
**Ontario M9V 1N8 (CA)**
- **SINGH, Jagbir**
**Vancouver**
**British Columbia V5X 2G1 (CA)**
- **GARG, Shyam Madhusudan**
**Richmond**
**British Columbia V6X 0M3 (CA)**

(74) Representative: **Venner, Julia Ann et al**
**Kilburn & Strode LLP**
**Lacon London**
**84 Theobalds Road**
**London WC1X 8NL (GB)**

(56) References cited:
**EP-A1- 0 523 189**
**WO-A1-2014/172045**
**US-A1- 2013 323 269**
**US-A1- 2016 022 580**
**US-A1- 2016 235 688**
**US-A1- 2016 250 354**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 519 578 B1

SEQUENCE LISTING

<110> Precision NanoSystems Inc.

<120> Compositions for Transfecting Resistant Cell Types

<130> PNI-IP-014PC

<150> 62/403,640 <151> 2016-10-03

<160> 6

<170> PatentIn version 3.5

<210> 1
<211> 966
<212> DNA
<213> Artificial

<220>
<223> mRNA eGFP ORF

<220>
<221> misc_feature
<222> (721)..(846)
<223> n is a, c, g, t or u

<400> 1

```
atggtgagca agggcgagga gctgttcacc ggggtggtgc ccatcctggt cgagctggac        60
ggcgacgtaa acggccacaa gttcagcgtg tccggcgagg gcgagggcga tgccacctac       120
ggcaagctga ccctgaagtt catctgcacc accggcaagc tgcccgtgcc ctggcccacc       180
ctcgtgacca ccctgaccta cggcgtgcag tgcttcagcc gctaccccga ccacatgaag       240
cagcacgact tcttcaagtc cgccatgccc gaaggctacg tccaggagcg caccatcttc       300
ttcaaggacg acggcaacta caagacccgc gccgaggtga agttcgaggg cgacaccctg       360
gtgaaccgca tcgagctgaa gggcatcgac ttcaaggagg acggcaacat cctggggcac       420
aagctggagt acaactacaa cagccacaac gtctatatca tggccgacaa gcagaagaac       480
ggcatcaagg tgaacttcaa gatccgccac aacatcgagg acggcagcgt gcagctcgcc       540
gaccactacc agcagaacac ccccatcggc gacggccccg tgctgctgcc cgacaaccac       600
tacctgagca cccagtccgc cctgagcaaa gaccccaacg agaagcgcga tcacatggtc       660
ctgctggagt tcgtgaccgc cgccgggatc actctcggca tggacgagct gtacaagtaa       720
nnnnnnnnnn nnnnnnnnnn nnnnnnnnnn nnnnnnnnnn nnnnnnnnnn nnnnnnnnnn       780
nnnnnnnnnn nnnnnnnnnn nnnnnnnnnn nnnnnnnnnn nnnnnnnnnn nnnnnnnnnn       840
nnnnnnaaaa aaaaaaaaaa aaaaaaaaaa aaaaaaaaaa aaaaaaaaaa aaaaaaaaaa       900
aaaaaaaaaa aaaaaaaaaa aaaaaaaaaa aaaaaaaaaa aaaaaaaaaa aaaaaaaaaa       960
aaaaaa                                                                  966
```

<210> 2
<211> 25
<212> DNA
<213> Artificial

<220>
<223> HPRT siRNA first strand

<400> 2
gccagacuuu guuggauuug aaatt 25

<210> 3
<211> 26
<212> DNA
<213> Artificial

<220>
<223> HPRT siRNA second strand

<400> 3
aauuucaauc caacaaaguc uggcuu 26

<210> 4
<211> 23
<212> DNA
<213> Artificial

<220>
<223> Sense strand for DsiNC-1 control

<400> 4
cguuaaucgc guauaauacg cgu 23

<210> 5
<211> 27
<212> DNA
<213> Artificial

<220>
<223> Antisense strand for DsiNC

<400> 5
auacgcguau uauacgcgau uaacgac 27

<210> 6
<211> 5514
<212> DNA
<213> Artificial

<220>
<223> Circular dsDNA pCX-EGFP

<400> 6

gtcgacattg attattgact agttattaat agtaatcaat tacggggtca ttagttcata 60

gcccatatat ggagttccgc gttacataac ttacggtaaa tggcccgcct ggctgaccgc 120

ccaacgaccc ccgcccattg acgtcaataa tgacgtatgt tcccatagta acgccaatag 180
ggactttcca ttgacgtcaa tgggtggact atttacggta aactgcccac ttggcagtac 240
atcaagtgta tcatatgcca agtacgcccc ctattgacgt caatgacggt aaatggcccg 300
cctggcatta tgcccagtac atgaccttat gggactttcc tacttggcag tacatctacg 360
tattagtcat cgctattacc atgggtcgag gtgagcccca cgttctgctt cactctcccc 420
atctcccccc cctccccacc cccaattttg tatttattta ttttttaatt attttgtgca 480
gcgatggggg cggggggggg gggggcgcgc gccaggcggg gcggggcggg gcgaggggcg 540
gggcggggcg aggcggagag gtgcggcggc agccaatcag agcggcgcgc tccgaaagtt 600
tccttttatg gcgaggcggc ggcggcggcg gccctataaa aagcgaagcg cgcggcgggc 660
gggagtcgct gcgttgcctt cgccccgtgc cccgctccgc gccgcctcgc gccgcccgcc 720
ccggctctga ctgaccgcgt tactcccaca ggtgagcggg cgggacggcc cttctcctcc 780
gggctgtaat tagcgcttgg tttaatgacg gctcgtttct tttctgtggc tgcgtgaaag 840
ccttaaaggg ctccgggagg gccctttgtg cggggggggag cggctcgggg ggtgcgtgcg 900
tgtgtgtgtg cgtggggagc gccgcgtgcg gcccgcgctg cccggcggct gtgagcgctg 960
cgggcgcggc gcggggcttt gtgcgctccg cgtgtgcgcg aggggagcgc ggccgggggc 1020
ggtgccccgc ggtgcggggg ggctgcgagg ggaacaaagg ctgcgtgcgg ggtgtgtgcg 1080
tgggggggtg agcagggggt gtgggcgcgg cggtcgggct gtaacccccc cctgcacccc 1140
cctccccgag ttgctgagca cggcccggct tcgggtgcgg ggctccgtac ggggcgtggc 1200
gcggggctcg ccgtgccggg cggggggtgg cggcaggtgg gggtgccggg cggggcgggg 1260
ccgcctcggg ccggggaggg ctcgggggag gggcgcggcg gcccccggag cgccggcggc 1320
tgtcgaggcg cggcgagccg cagccattgc cttttatggt aatcgtgcga gagggcgcag 1380
ggacttcctt tgtcccaaat ctgtgcggag ccgaaatctg ggaggcgccg ccgcaccccc 1440
tctagcgggc gcggggcgaa gcggtgcggc gccggcagga aggaactggg cggggagggc 1500
cttcgtgcgt cgccgcgccg ccgtcccctt ctccctctcc agcctcgggg ctgtccgcgg 1560
ggggacggct gccttcgggg gggacggggc agggcggggt tcggcttctg gcgtgtgacc 1620
ggcggctcta gagcctctgc taaccatgtt catgccttct tcttttttcct acagctcctg 1680
ggcaacgtgc tggttattgt gctgtctcat cattttggca aagaattcgc caccatggtg 1740
agcaagggcg aggagctgtt caccggggtg gtgcccatcc tggtcgagct ggacggcgac 1800
gtgaacggcc acaagttcag cgtgtccggc gagggcgagg gcgatgccac ctacggcaag 1860
ctgaccctga agttcatctg caccaccggc aagctgcccg tgccctggcc caccctcgtg 1920
accaccctga cctacggcgt gcagtgcttc agccgctacc ccgaccacat gaagcagcac 1980
gacttcttca agtccgccat gcccgaaggc tacgtccagg agcgcaccat cttcttcaag 2040

gacgacggca actacaagac ccgcgccgag gtgaagttcg agggcgacac cctggtgaac 2100
cgcatcgagc tgaagggcat cgacttcaag gaggacggca acatcctggg gcacaagctg 2160
gagtacaact acaacagcca caacgtctat atcatggccg acaagcagaa gaacggcatc 2220
aaggtgaact tcaagatccg ccacaacatc gaggacggca gcgtgcagct cgccgaccac 2280
taccagcaga acacccccat cggcgacggc cccgtgctgc tgcccgacaa ccactacctg 2340
agcacccagt ccgccctgag caaagacccc aacgagaagc gcgatcacat ggtcctgctg 2400
gagttcgtga ccgccgccgg gatcactcac ggcatggacg agctgtacaa gtaagaattc 2460
actcctcagg tgcaggctgc ctatcagaag gtggtggctg gtgtggccaa tgccctggct 2520
cacaaatacc actgagatct tttccctct gccaaaaatt atggggacat catgaagccc 2580
cttgagcatc tgacttctgg ctaataaagg aaatttattt tcattgcaat agtgtgttgg 2640
aattttttgt gtctctcact cggaaggaca tatgggaggg caaatcattt aaaacatcag 2700
aatgagtatt tggtttagag tttggcaaca tatgccatat gctggctgcc atgaacaaag 2760
gtggctataa agaggtcatc agtatatgaa acagccccct gctgtccatt ccttattcca 2820
tagaaaagcc ttgacttgag gttagatttt ttttatattt tgttttgtgt tatttttttc 2880
tttaacatcc ctaaaatttt ccttacatgt tttactagcc agatttttcc tcctctcctg 2940
actactccca gtcatagctg tccctcttct cttatgaaga tccctcgacc tgcagcccaa 3000
gcttggcgta atcatggtca tagctgtttc ctgtgtgaaa ttgttatccg ctcacaattc 3060
cacacaacat acgagccgga agcataaagt gtaaagcctg gggtgcctaa tgagtgagct 3120
aactcacatt aattgcgttg cgctcactgc ccgctttcca gtcgggaaac ctgtcgtgcc 3180
agcggatccg catctcaatt agtcagcaac catagtcccg cccctaactc cgcccatccc 3240
gcccctaact ccgcccagtt ccgcccattc tccgccccat ggctgactaa ttttttttat 3300
ttatgcagag gccgaggccg cctcggcctc tgagctattc cagaagtagt gaggaggctt 3360
ttttggaggc ctaggctttt gcaaaaagct aacttgttta ttgcagctta taatggttac 3420
aaataaagca atagcatcac aaatttcaca aataaagcat ttttttcact gcattctagt 3480
tgtggtttgt ccaaactcat caatgtatct tatcatgtct ggatccgctg cattaatgaa 3540
tcggccaacg cgcggggaga ggcggtttgc gtattgggcg ctcttccgct tcctcgctca 3600
ctgactcgct gcgctcggtc gttcggctgc ggcgagcggt atcagctcac tcaaaggcgg 3660
taatacggtt atccacagaa tcaggggata acgcaggaaa gaacatgtga gcaaaaggcc 3720
agcaaaaggc caggaaccgt aaaaaggccg cgttgctggc gtttttccat aggctccgcc 3780
cccctgacga gcatcacaaa aatcgacgct caagtcagag gtggcgaaac ccgacaggac 3840
tataaagata ccaggcgttt ccccctggaa gctccctcgt gcgctctcct gttccgaccc 3900

```
tgccgcttac cggatacctg tccgcctttc tcccttcggg aagcgtggcg ctttctcaat      3960
gctcacgctg taggtatctc agttcggtgt aggtcgttcg ctccaagctg ggctgtgtgc      4020
acgaaccccc cgttcagccc gaccgctgcg ccttatccgg taactatcgt cttgagtcca      4080
acccggtaag acacgactta tcgccactgg cagcagccac tggtaacagg attagcagag      4140
cgaggtatgt aggcggtgct acagagttct tgaagtggtg gcctaactac ggctacacta      4200
gaaggacagt atttggtatc tgcgctctgc tgaagccagt taccttcgga aaaagagttg      4260
gtagctcttg atccggcaaa caaaccaccg ctggtagcgg tggttttttt gtttgcaagc      4320
agcagattac gcgcagaaaa aaaggatctc aagaagatcc tttgatcttt tctacggggt      4380
ctgacgctca gtggaacgaa aactcacgtt aagggatttt ggtcatgaga ttatcaaaaa      4440
ggatcttcac ctagatcctt ttaaattaaa aatgaagttt taaatcaatc taaagtatat      4500
atgagtaaac ttggtctgac agttaccaat gcttaatcag tgaggcacct atctcagcga      4560
tctgtctatt tcgttcatcc atagttgcct gactccccgt cgtgtagata actacgatac      4620
gggagggctt accatctggc cccagtgctg caatgatacc gcgagaccca cgctcaccgg      4680
ctccagattt atcagcaata aaccagccag ccggaagggc cgagcgcaga agtggtcctg      4740
caactttatc cgcctccatc cagtctatta attgttgccg ggaagctaga gtaagtagtt      4800
cgccagttaa tagtttgcgc aacgttgttg ccattgctac aggcatcgtg gtgtcacgct      4860
cgtcgtttgg tatggcttca ttcagctccg gttcccaacg atcaaggcga gttacatgat      4920
cccccatgtt gtgcaaaaaa gcggttagct ccttcggtcc tccgatcgtt gtcagaagta      4980
agttggccgc agtgttatca ctcatggtta tggcagcact gcataattct cttactgtca      5040
tgccatccgt aagatgcttt tctgtgactg gtgagtactc aaccaagtca ttctgagaat      5100
agtgtatgcg gcgaccgagt tgctcttgcc cggcgtcaat acgggataat accgcgccac      5160
atagcagaac tttaaaagtg ctcatcattg gaaaacgttc ttcggggcga aaactctcaa      5220
ggatcttacc gctgttgaga tccagttcga tgtaacccac tcgtgcaccc aactgatctt      5280
cagcatcttt tactttcacc agcgtttctg ggtgagcaaa aacaggaagg caaaatgccg      5340
caaaaaaggg aataagggcg acacggaaat gttgaatact catactcttc ctttttcaat      5400
attattgaag catttatcag ggttattgtc tcatgagcgg atacatattt gaatgtattt      5460
agaaaaataa acaaataggg gttccgcgca catttccccg aaaagtgcca cctg            5514
```

## Description

This application claims the benefit of United States application number 62/403,640 filed on October 3, 2016.

## BACKGROUND OF THE INVENTION

### Field of Invention

The field of the invention is the transfer of active nucleic acids into cells.

### Related art

Nucleic acids in the form of polynucleotides or oligonucleotides can be used to focus treatment on a particular genetic target, either by interfering with its expression, or by restoring or augmenting its expression, or by editing the gene.
Delivering nucleic acids into cells or tissues presents challenges because nucleic acids are relatively large, negatively charged, hydrophilic compounds which are not capable of passively diffusing across the cell membrane and are also vulnerable to nucleases.(Akhtar, Basu S Fau - Wickstrom et al. 1991)
Existing approaches for delivering these nucleic acids across the cell membrane include viruses such as adeno-associated viruses as vectors for gene restoration, but these can cause immune responses in treated individuals(Mingozzi and High 2013). Cationic lipids and polymers have also been used in experiments, but each has issues of transfection efficiency, stability and toxicity.(Lv, Zhang et al. 2006) To increase the therapeutic activity of the nucleic acids, significant efforts in the field have focused on lipid nanoparticles (LNP) that comprise cationic lipids, including ionizable cationic lipids (also known as "cationic lipids") and PEGylated lipids, for the efficient encapsulation and delivery of nucleic acids to cells(Tam, Chen et al. 2013, Kauffman, Webber et al. 2015). LNPs have been engineered to obtain different pharmacokinetics, different biodistribution in tissues, biodegradability, or altered toxicity, to favor the therapeutic activity of the nucleic acid.
CNS tissue is notoriously difficult to transfect (O'Mahony, Godinho et al. 2013). The cells are sensitive to their conditions and are hard to maintain *in vitro.*
WO 2014/172045 A1 discloses a transfection reagent composition comprising 1, 17-bis(2-octylcyclopropyl)heptadecan-9-yl-4-(dimethylamino)butanotanoate as cationic lipid, a structural lipid which may be DOPE, cholesterol and a stabilizing agent.
A more effective method for effectively delivering nucleic acids into neurons is still required.

## SUMMARY OF THE INVENTION

Aspects of the invention are set out in the independent claims. Particular embodiments of these aspects are set out in the dependent claims. Any subject matter contained herein that does not fall within the scope of the appended claims is considered as being useful for understanding the invention.
Also disclosed herein is a transfection reagent composition comprising: 30-60 MOL% of an cationic lipid, or pharmaceutical acceptable salt thereof; 10-60 MOL% structural lipid; a sterol and 0.1 to about 10MOL% Stabilizing agent. The cationic lipid may be an amino lipid. The cationic lipid may be selected from the group consisting of 1,17-bis(2-octylcyclopropyl)heptadecan-9-yl-4-(dimethylamino)butanoate, DODAC, DOTMA, DDAB, DOTAP, DOTAP·Cl, DC-Chol, DOSPA, DOGS, DODAP, DODMA, DMRIE, C12-200, and pharmaceutically acceptable salts thereof. The cationic lipid may have the formula:

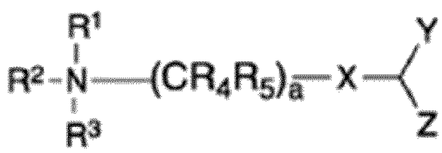

or a pharmaceutically acceptable salt thereof, wherein:

R1 and R2 are each independently H, alkyl, akenyl, alkynyl, cycloalkyl, aryl, heteroaryl, and heterocyclyl, wherein each of alkyl, alkenyl, alkynyl, cycloalkyl, aryl, heteroaryl, and heterocyclyl is optionally substituted by H, halo, hydroxy, cyano, oxo, Ci-C6 alkyl optionally substituted by halo, hydroxy, or alkoxy;
or $R_1$ and $R_2$ are taken together with the N atom to which they are both attached to form a 3-8 member heteroaryl or heterocyclyl;

wherein each of the heteroaryl and heterocyclyl is optionally substituted by H, halo, hydroxy, cyano, oxo, nitro, $C_1$-$C_6$ alkyl optionally substituted by halo, hydroxyl, or alkoxy;

$R_3$ is absent, H, alkyl, alkenyl, alkynyl, cycloalkyl, aryl, heteroaryl, or heterocyclyl;

$R_4$ and $R_5$ are each independently H, alkyl, alkenyl, alkynyl, cycloalkyl, aryl, heteroaryl, or heterocyclyl;

wherein each of alkyl, alkenyl, alkynyl, cycloalkyl, aryl, heteroaryl, and heterocyclyl is optionally substituted by H, halo, hydroxy; cyano; oxo; $C_1$-$C_6$ alkyl optionally substituted by halo, hydroxy, or alkoxy;

X is O, S, -$NR_4$-, -S-S-, -OC(=0)-, -C(=0)0-, -OC(=0)0-, -$NR_4$C(=0)-, -C(=0)$NR_4$-, -$NR_4$C(=0)0-, - OC(=0)$NR_4$-, -$NR_4$C $NR_4$, -$NR_4$C(=S)0-, -OC(=S)$NR_4$-, -$NR_4$C(=S)$NR_4$-, or -$CR_4R_5$-;

Y and Z are independently $C_{10}$ to $C_{30}$ groups having the formula $L_1$-($CR_6$R7)a-[L2-($CR_6R_7$)P]y-$L_3$- Re, wherein: $L_1$ is a bond, -($CR_6$Ry)-, -0-, -CO-, -$NR_8$-, -S-, or a combination thereof; each 5 and $R_7$, independently, is H, halo, hydroxyl, cyano, $C_1$-$C_6$ alkyl optionally substituted by halo, hydroxyl, or alkoxy;

$L_2$ is a bond, -($CR_6R_7$)-, -0-, -CO-, -$NR_8$-, -S-,

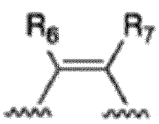 , 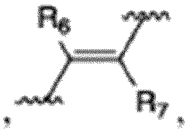 , 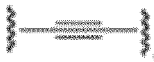 ,

or a combination thereof, or has the formula

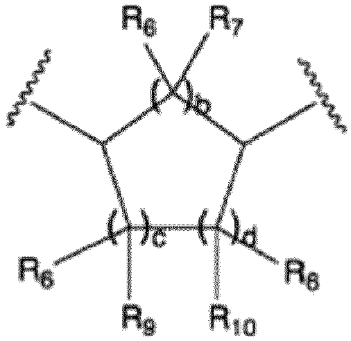

wherein b, c, and d are each independently 0, 1, 2, or 3, given the sum of b, c, and d is at least 1 and no greater than 8; and $R_9$ and $R_{10}$ are each independently $R_7$, or adjacent $R_9$ and $R_{10}$, taken together, are optionally a bond;

$L_3$ is a bond, -($CR_6R_7$)-, -0-, -CO-, -$NR_8$-, -S-,

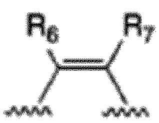 , 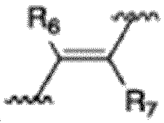

or a combination thereof;

$R_8$ is independently H, halo, hydroxy, cyano, alkoxy, aryl, heteroaryl, or $C_1$-$C_6$ alkyl optionally substituted by halo, hydroxy, or heterocyclyl, or $R_8$ has the formula:

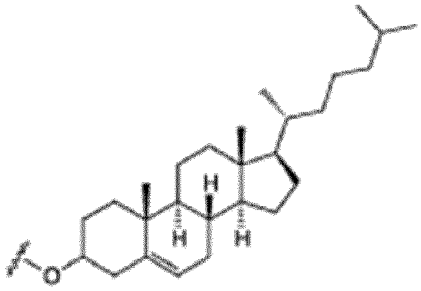

a is 0, 1 , 2, 3, or 4; a is 0-6; each β, independently, is 0-6; and y is 0-6.

The cationic lipid of the present invention is 1,17-bis(2-octylcyclopropyl)heptadecan-9-yl-4-(dimethylamino)butanoate or a pharmaceutically acceptable salt thereof.

The structural lipid may be selected from the group consisting of diacylphosphatidylcholines, diacylphosphatidylethanolamines, sterols, ceramides, sphingomyelins, dihydrosphingomyelins, cephalins, and cerebrosides. According to the invention, the structural lipid is 1,2-dioleoyl-sn-glycero-3-phosphoethanolamine (DOPE).

The stabilizing agent may be selected from the group consisting of polyethylene glycol, polyethylene glycol-DMG, polyoxyethylene alkyl ethers, diblock polyoxyethylene ether co-polymers, triblock polyoxyethylene alkyl ethers co-polymers, and amphiphilic branched polymers.

According to the invention, the sterol is cholesterol. The stabilizing agent may be selected from the group consisting of polyoxyethylene (20) oleyl ether, polyoxyethylene (23) lauryl ether, polyoxyethylene (40) stearate, poly(propylene glycol)11-block-poly(ethylene glycol)16-block-poly(propylene glycol)11, poly(propylene glycol)12-block-poly(ethylene glycol)28-block-poly(propylene glycol)12. The stabilizing agent may be PEG-conjugated lipid. The stabilizing agent may be PEG-DMG. The cationic lipid, according to the invention, comprises 40MOL%. The stabilizing agent comprises 2.5MOL% stabilizer.

Also described, but not claimed, is a transfection reagent composition comprising: 30-60 MOL% of an cationic lipid, or pharmaceutical acceptable salt thereof; 10-60 MOL% structural lipid; a sterol and 0.1 to about 10MOL% Stabilizing agent. wherein: the cationic lipid is 1,17-bis(2-octylcyclopropyl)heptadecan-9-yl-4-(dimethylamino)butanoate or a pharmaceutically acceptable salt thereof; the structural lipid is 1,2-dioleoyl-sn-glycero-3-phosphoethanolamine (DOPE); the sterol is cholesterol, and the stabilizing agent is polyoxyethylene (40) stearate.

Also described, but not claimed, is a transfection reagent composition comprising: 30-60 MOL% of an cationic lipid, or pharmaceutical acceptable salt thereof; 10-60 MOL% structural lipid; a sterol and 0.1 to about 10MOL% Stabilizing agent. wherein: the cationic lipid is 1,17-bis(2-octylcyclopropyl)heptadecan-9-yl-4-(dimethylamino)butanoate or a pharmaceutically acceptable salt thereof; the structural lipid is 1,2-dioleoyl-sn-glycero-3-phosphoethanolamine (DOPE); the sterol is cholesterol, and the stabilizing agent is polyethylene glycol conjugated lipid.

The structural lipid comprises 10 to about 40MOL% 1,2-dioleoyl-sn-glycero-3-phosphoethanolamine (DOPE). The sterol is present at 10 to 20MOL%.

The composition may further include a nucleic acid. The nucleic acid may be a therapeutic nucleic acid. In other examples, it is a restorative nucleic acid replacing a missing or poorly functioning gene in a cell. In other examples, it is a modulating nucleic acid that reduces a genetic action in the cell. In other examples, it is a diagnostic nucleic acid, that identifies diseased cells.

The nucleic acid may be a DNA, an RNA, a locked nucleic acid, a nucleic acid analog, or a plasmid capable of expressing an RNA.

The nucleic acid may be an antisense oligonucleotide, ribozyme, miRNA, rRNA, tRNA, siRNA, saRNA, snRNA, snoRNA, IncRNA, piRNA, tsRNA, srRNA, crRNA, tracrRNA, sgRNA, shRNA, ncRNA, mRNA, pre-condensed DNA, pDNA, an aptamer, or a combination thereof. The transfecting reagent may be used to transfect a neuron, an astrocyte, and/or a progenitor cell. The composition may exist in the form of nanoparticles having a diameter of from about 15nm, 20nm, 30nm, 40nm, 50nm, 60nm, 70nm, 80nm, 90nm, 100nm, 110nm, 120nm, 130nm, 140nm, 150nm, 160nm, 170nm, 180nm, 190nm, 200nm, to about 300nm.

Also described is a method for introducing a nucleic acid into a cell, by contacting a cell with a transfecting reagent described above in combination with a nucleic acid, and wherein the activity of the nucleic acid, as well as the viability of the cell, are maintained. There is also provided a method for modulating the expression of a target polynucleotide or polypeptide, comprising contacting a cell with the transfecting reagent in combination with a nucleic acid, wherein the nucleic acid is capable of modulating the expression of a target polynucleotide or polypeptide while the viability of the cell is preserved. The cell may be a neuron. The cell may be an astrocyte. The cell may be a progenitor cell.

There is also provided a method of manufacturing a lipid nanoparticle capable of targeting neurons, the method including increasing the ratio of cationic lipid to structural lipid in the lipid nanoparticle.

There is also provided a method of manufacturing a lipid nanoparticle capable of targeting astrocytes, the method including decreasing the ratio of cationic lipid to structural lipid in the lipid nanoparticle. The cell may be a mammalian cell. The cell may be human.

Other aspects and features of the present disclosure and invention will become apparent to those ordinarily skilled in the art upon review of the following description of specific embodiments of the disclosure and invention in conjunction with the accompanying figures.

BRIEF DESCRIPTION OF THE DRAWINGS

**In drawings which illustrate embodiments of the invention,**

Figure 1 is a photograph of neurons that have been treated with labelled Lipid Mix D formulated nanoparticles containing GFP mRNA. The top right is MAP2 (a neuronal marker) antibody staining, the bottom right is DiD labelling of the nanoparticle (a lipid label); the bottom left is GFP expression in the neurons, and the top left quadrant is a merged image showing DiD, MAP2, and GFP, establishing GFP expression in the live neurons;

Figure 2 is a photograph showing staining perspectives to illustrate the ability of Lipid Mix B to transfect primary mixed neuronal culture. The top right is MAP2 (a neuronal marker) antibody staining, the bottom right is DiD labelling of the nanoparticle (a lipid label); the bottom left is GFP expression in the neurons, and the top left quadrant is a merged image showing DiD, DAPI, MAP2, and GFP, establishing GFP expression occurs in the glial cells. All images include DAPi, a nuclear stain;

Figure 3 is a photograph showing staining perspectives to illustrate the ability of Lipid Mix C to transfect, and the viability of cells. The top right is MAP2 antibody staining, the bottom right is DID labelling of the nanoparticle; the bottom left is GFP expression in the neurons, and the top left quadrant is a merged image showing DiD, DAPI, MAP2, and GFP, establishing GFP expression in the live neurons. All images include DAPi, a nuclear stain;

Figure 4 is a bar graph showing the level of GFP expression in neurons measured by ELISA following treatment with mRNA GFP Lipid Mix A and Lipid Mix D nanoparticles;

Figure 5 is a bar graph showing the mean fluorescence intensity of GFP expression levels in neurons following treatment with mRNA GFP for Lipid Mix A and Lipid Mix D in comparison with Lipofectamine™ Messenger Max™ transfecting agent;

Figure 6 is a bar graph showing viability of astrocytes 48 hours post treatment with mRNA loaded in Lipid Mix D versus MessengerMax™ transfecting reagent, as measured by Presto Blue assay;

Figure 7 is a flow cytometry histogram showing Lipid Mix D GFP mRNA LNP-mediated mean fluorescence intensity in human neural progenitor cells (grey histogram) compared to untreated cells (black histogram)

Figure 8 are two bar graphs showing Mean Fluorescent Intensity (MFI) levels and Percentage (%) of cells expressing GFP by flow cytometry of neural progenitor cells treated with GFP plasmid in LNPs comprised of Lipid Mix B (50 MOL% cationic lipid) and Lipid Mix C (40 MOL% cationic lipid);

Figure 9 are two bar graphs showing percentage (%) of cells expressing GFP and Mean Fluorescent Intensity (MFI) levels via flow cytometry for neural progenitor cells treated with Lipid Mix A and Lipid Mix D GFP plasmid LNPs 48 hours post exposure;

Figure 10 is a bar graph showing Mean Flourescence Intensity of GFP expression in human neural progenitor cells treated with GFP plasmid LNP formulations of Lipid Mix A, , Lipid Mix B, Lipid Mix C, Lipid Mix D , Lipid Mix E and Lipid Mix F by flow cytometry.

## DETAILED DESCRIPTION

The present disclosure and invention provides compositions, lipid particles containing a therapeutic agent, methods for making the lipid particles containing a therapeutic agent, methods for targeting specific cell types, and methods for delivering a therapeutic agent using the lipid particles.

Described herein are compositions of matter including one or more cationic lipid(s), one or more structural lipid(s), and one or more stabilizing agent(s).

The compositions of the invention may be provided for mixing with nucleic acid therapeutics for delivery to target cells or tissues, or for treatment of mammals in need of such delivery for treatment of insufficiency or disease. Methods of treatment are disclosed herein but do not form part of the claimed invention.

Also described are compositions of matter including one or more cationic lipid(s), one or more structural lipid(s), one or more stabilizing agent(s), and one or more nucleic acid (NA).

The compositions may be provided for formulating nucleic acid therapeutics for the treatment of diseases of the CNS. Also described is a method for changing the target of a lipid nanoparticle from astrocytes to neurons.

"Lipid" refers to a group of organic compounds that are esters of fatty acids and are characterized by being insoluble in water but soluble in many organic solvents.

Lipid Particles. The invention provides lipid particles manufactured from the compositions described above. The lipid particles contain a therapeutic agent in some embodiments. The lipid particles include one or more cationic lipids, one or more structural lipid(s), one or more stabilizing agent(s), and one or more nucleic acids.

Cationic lipid. The lipid particles include a cationic lipid. As used herein, the term "cationic lipid" refers to a lipid that is cationic or becomes ionizable (protonated) as the pH is lowered below the pK of the cationic group of the lipid, but is progressively more neutral at higher pH values. At pH values below the pK, the lipid is then able to associate with negatively charged nucleic acids (e.g., oligonucleotides). As used herein, the term "cationic lipid" includes zwitterionic lipids that assume a positive charge on pH decrease, and any of a number of lipid species which carry a net positive

charge at a selective pH, such as physiological pH. Such lipids include, but are not limited to, N,N-dioleyl-N,N-dimethylammonium chloride (DODAC); N-(2,3-dioleyloxy)propyl)-N,N,N-trimethylammonium chloride (DOTMA); N,N-distearyl-N,N-dimethylammonium bromide (DDAB); N-(2,3-dioleoyloxy)propyl)-N,N,N-trimethylammonium chloride (DOTAP); 3-(N— (N',N'-dimethylaminoethane)-carbamoyl)cholesterol (DC-Chol), C12-200 and N-(1,2-dimyristyloxyprop-3-yl)-N,N-dimethyl-N-hydroxyethyl ammonium bromide (DMRIE).

The cationic lipid may be an amino lipid. Suitable amino lipids useful in the disclosure and invention include those described in WO 2009/096558. Representative amino lipids include 1,17-bis(2-octylcyclopropyl)heptadecan-9-yl 4-(dimethylamino)butanoate hydrochloride, I,2-dilinoleyoxy-3-(dimethylamino)acetoxypropane (DLin-DAC), 1,2-dilinoleyoxy-3-morpholinopropane (DLin-MA), 1,2-dilinoleoyl-3-dimethylaminopropane (DLinDAP), I,2-dilinoleylthio-3-dimethylaminopropane (DLin-S-DMA), I-linoleoyl-2-linoleyloxy-3-dimethylaminopropane (DLin-2-DMAP), I,2-dilinoleyloxy-3-trimethylaminopropane chloride salt (DLin-TMA·Cl), I,2-dilinoleoyl-3-trimethylaminopropane chloride salt (DLin-TAP·Cl), I,2-dilinoleyloxy-3-(N-methylpiperazino)propane (DLin-MPZ), 3-(N,N-dilinoleylamino)-I,2-propanediol (DLinAP), 3-(N,N-dioleylamino)-I,2-propanedio (DOAP), 1,2-dilinoleyloxo-3-(2-N,N-dimethylamino)ethoxypropane (DLin-EG-DMA), and 2,2-dilinoleyl-4-dimethylaminomethyl-[1,3]-dioxolane (DLin-K-DMA).

Suitable amino lipids include those having the general formula:

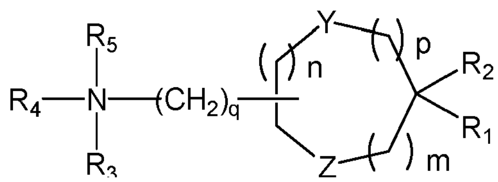

wherein $R_1$ and $R_2$ are either the same or different and independently optionally substituted $C_{10}$-$C_{24}$ alkyl, optionally substituted $C_{10}$-$C_{24}$ alkenyl, optionally substituted $C_{10}$-$C_{24}$ alkynyl, or optionally substituted $C_{10}$-$C_{24}$ acyl;
$R_3$ and $R_4$ are either the same or different and independently optionally substituted $C_1$-$C_6$ alkyl, optionally substituted $C_2$-$C_6$ alkenyl, or optionally substituted $C_2$-$C_6$ alkynyl or $R_3$ and $R_4$ may join to form an optionally substituted heterocyclic ring of 4 to 6 carbon atoms and 1 or 2 heteroatoms chosen from nitrogen and oxygen;
$R_5$ is either absent or present and when present is hydrogen or $C_1$-$C_6$ alkyl;
m, n, and p are either the same or different and independently either 0 or 1 with the proviso that m, n, and p are not simultaneously 0;
q is 0, 1, 2, 3, or 4; and Y and Z are either the same or different and independently O, S, or NH.

In one example, $R_1$ and $R_2$ are each linoleyl, and the amino lipid is a dilinoleyl amino lipid. The amino lipid may be a dilinoleyl amino lipid. According to the invention, the cationic lipid is 1,17-bis(2-octylcyclopropyl)heptadecan-9-yl-4-(dimethylamino)butanoate, or a pharmaceutically acceptable salt thereof.

In embodiments of the invention, the cationic lipid is 1,17-bis(2-octylcyclopropyl)heptadecan-9-yl 4-(dimethylamino)butanoate hydrochloride. This compound is disclosed in United States Published Application 2013323269.

In other examples of the disclosure, the cationic lipid-like material is C12-200 as described by Kaufmann and his colleagues (Kaufmann k 2015)

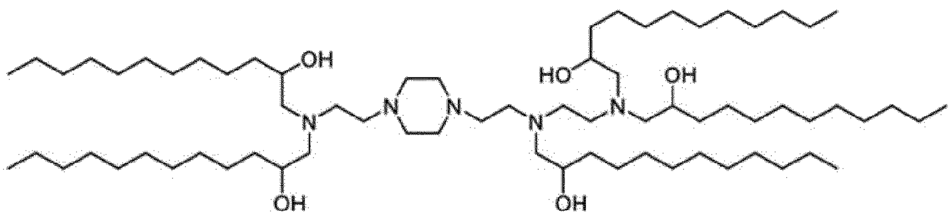

The cationic lipid is present in examples of the composition and may be comprised in an amount from about 30 to about 60 mole percent ("MOL%", or the percentage of the total moles that is of a particular component), preferably from 30 to 50MOL%. The cationic lipid may be present in 35MOL%. In the examples which are according to the invention, the cationic lipid is present in 40MOL%.

Structural lipids. The composition and lipid particles of the disclosure and invention include one or more structural lipids. Suitable structural lipids support the formation of particles during manufacture. Structural lipids refer to any one of a number of lipid species that exist in either in an anionic, uncharged or neutral zwitterionic form at physiological pH. Representative structural lipids include diacylphosphatidylcholines, diacylphosphatidylethanolamines, diacylphosphatidylglycerols, ceramides, sphingomyelins, dihydrosphingomyelins, cephalins, and cerebrosides.

Exemplary structural lipids include zwitterionic lipids, for example, distearoylphosphatidylcholine (DSPC), dioleoylphosphatidylcholine (DOPC), dipalmitoylphosphatidylcholine (DPPC), dioleoyl-phosphatidylethanolamine (DOPE), palmitoyloleoylphosphatidylcholine (POPC), palmitoyloleoyl-phosphatidylethanolamine (POPE) and dioleoyl-phosphatidylethanolamine 4-(N-maleimidomethyl)-cyclohexane-1-carboxylate (DOPE-mal), dipalmitoyl phosphatidyl ethanolamine (DPPE), dimyristoylphosphoethanolamine (DMPE), distearoyl-phosphatidylethanolamine (DSPE), 16-O-monomethyl PE, 16-O-dimethyl PE, 18-1-trans PE, 1-stearioyl-2-oleoyl-phosphatidyethanol amine (SOPE), and 1,2-dielaidoyl-sn-glycero-3-phophoethanolamine (transDOPE).

In the invention, the structural lipid is 1,2-dioleoyl-sn-glycero-3-phosphoethanolamine (DOPE).

The structural lipid may be any lipid that is negatively charged at physiological pH. These lipids include phosphatidylglycerol such as dioleoylphosphatidylglycerol (DOPG), dipalmitoylphosphatidylglycerol (DPPG), palmitoyloleyolphosphatidylglycerol (POPG), cardiolipin, phosphatidylinositol, diacylphosphatidylserine, diacylphosphatidic acid, other anionic modifying groups joined to neutral lipids.

Stabilizing Agents are included in compositions and lipid nucleic acid examples and embodiments to ensure integrity of the mixture. Stabilizing agents may be polyethylene glycol-lipids. Suitable polyethylene glycol-lipids include PEG-modified phosphatidylethanolamine, PEG-modified phosphatidic acid, PEG-modified ceramides (e.g., PEG-CerC14 or PEG-CerC20), PEG-modified dialkylamines, PEG-modified diacylglycerols, PEG-modified dialkylglycerols. Representative polyethylene glycol-lipids include PEG-c-DOMG, PEG-c-DMA, and PEG-s-DMG. In one embodiment, the polyethylene glycol-lipid is N-[(methoxy poly(ethylene glycol)$_{2000}$)carbamyl]-1,2-dimyristyloxlpropyl-3-amine (PEG-c-DMA).

The polyethylene glycol-lipid may be PEG-c-DOMG. The polyethylene glycol-lipid may be PEG-DMG (1,2-Dimyristoyl-sn-glycerol, methoxypolyethylene glycol). The polyethylene glycol lipid content may be from 0 to 10%

Sterols are included in the claimed compositions, and lipid particles made therefrom include cholesterol.

Cholesterol was present 37 or 17 MOL% in some examples and embodiments, and 0 MOL% in one formulation tested. Stabilizing agent was present at 1 to 2.5 MOL%. According to the invention, the stabilizing agent is present at 2.5 MOL%.

In examples of the invention, the cationic lipid 1,17-bis(2-octylcyclopropyl)heptadecan-9-yl 4-(dimethylamino)butanoate hydrochloride, was varied from 35MOL% to 50 MOL% while keeping the structural lipid (DSPC, DOPC, DOPE, POPC, for example) at 10 to 40 MOL%.

Other suitable structural lipids may include glycolipids (e.g., monosialoganglioside $GM_1$).

According to the invention, the structural lipid is present in the lipid particle in an amount from 10 to 40 MOL%. The structural lipid may be present in the lipid particle in an amount from about 20 to about 30 MOL%. The structural lipid may be present in the lipid particle in about 17MOL%. The structural lipid may be present in the lipid particle in about 20 MOL%, or in about 30MOL%, or in about 40MOL%.

Nucleic Acids. The compositions and lipid particles of the present invention are useful for the systemic or local delivery of nucleic acid therapeutics. As described herein, the nucleic acid therapeutic (NAT)is incorporated into the lipid particle during its formation. As used herein, the term "nucleic acid therapeutic" (NAT) is meant to include any oligonucleotide or polynucleotide whose delivery into a cell causes a desirable effect Fragments containing up to 50 nucleotides are generally termed oligonucleotides, and longer fragments are called polynucleotides. In particular embodiments, oligonucleotides of the present invention are 20-50 nucleotides in length. Embodiments of the invention, oligonucleotides are 996 to 4500 nucleotides in length, as in the case of messenger RNA. In the context of this invention, the terms "polynucleotide" and "oligonucleotide" refer to a polymer or oligomer of nucleotide or nucleoside monomers consisting of naturally occurring bases, sugars and intersugar (backbone) linkages. The terms "polynucleotide" and "oligonucleotide" also includes polymers or oligomers comprising non-naturally occurring monomers, or portions thereof, which function similarly. Such modified or substituted oligonucleotides are often preferred over native forms because of properties such as, for example, enhanced cellular uptake and increased stability in the presence of nucleases. Oligonucleotides are classified as deoxyribooligonucleotides or ribooligonucleotides. A deoxyribooligonucleotide consists of a 5-carbon sugar called deoxyribose joined covalently to phosphate at the 5' and 3' carbons of this sugar to form an alternating, unbranched polymer. A ribooligonucleotide consists of a similar repeating structure where the 5-carbon sugar is ribose. The nucleic acid that is present in a lipid particle according to this invention includes any form of nucleic acid that is known. The nucleic acids used herein can be single-stranded DNA or RNA, or double-stranded DNA or RNA, or DNA-RNA hybrids. Examples of double-stranded DNA include structural genes, genes including control and termination regions, and self-replicating systems such as viral or plasmid DNA. Examples of double-stranded RNA include siRNA and other RNA interference reagents. Single-stranded nucleic acids include antisense oligonucleotides, ribozymes, microRNA, mRNA, and triplex-forming oligonucleotides.

In one embodiment, the polynucleic acid is an antisense oligonucleotide. In certain embodiments, the nucleic acid is a ribozyme, a non-coding nuclear or nucleolar RNA as explained in HUGO http://www.genenames.org/cgi-bin/genefamilies, miRNA, rRNA, tRNA, siRNA, saRNA, snRNA, snoRNA, lncRNA, piRNA, tsRNA, srRNA, crRNA, tracrRNA, sgRNA, shRNA, ncRNA, mRNA, pre-condensed DNA, pDNA, an aptamer, or a combination thereof. In one embodiment,

the nucleic acid therapeutic (NAT) is a plasmid or circular nucleic acid construct. In one embodiment, the NAT is a mRNA. In one embodiment, the NAT is a siRNA. In one embodiment, the NAT is a miRNA. In one embodiment, the NAT is a tracrRNA. In one embodiment, the NAT is a sgRNA.

The term "nucleic acids" also refers to ribonucleotides, deoxynucleotides, modified ribonucleotides, modified deoxyribonucleotides, modified phosphate-sugar-backbone oligonucleotides, other nucleotides, nucleotide analogs, and combinations thereof, and can be single stranded, double stranded, or contain portions of both double stranded and single stranded sequence, as appropriate.

The term "nucleotide," as used herein, generically encompasses the following terms, which are defined below: nucleotide base, nucleoside, nucleotide analog, and universal nucleotide.

The term "nucleotide base," as used herein, refers to a substituted or unsubstituted parent aromatic ring or rings. In some Embodiments, the aromatic ring or rings contain at least one nitrogen atom. In some embodiments, the nucleotide base is capable of forming Watson-Crick and/or Hoogsteen hydrogen bonds with an appropriately complementary nucleotide base. Exemplary nucleotide bases and analogs thereof include, but are not limited to, purines such as 2-aminopurine, 2,6-diaminopurine, adenine (A), ethenoadenine, N6-2-isopentenyladenine (6iA), N6-2-isopentenyl-2-methylthioadenine (2ms6iA), N6-methyladenine, guanine (G), isoguanine, N2-dimethylguanine (dmG), 7-methylguanine (7mG), 2-thiopyrimidine, 6-thioguanine (6sG) hypoxanthine and O6-methylguanine; 7-deaza-purines such as 7-deazaadenine (7-deaza-A) and 7-deazaguanine (7-deaza-G); pyrimidines such as cytosine (C), 5-propynylcytosine, isocytosine, thymine (T), 4-thiothymine (4sT), 5,6-dihydrothymine, O4-methylthymine, uracil (U), 4-thiouracil (4sU) and 5,6-dihydrouracil (dihydrouracil; D); indoles such as nitroindole and 4-methylindole; pyrroles such as nitropyrrole; nebularine; base (Y); In some Embodiments, nucleotide bases are universal nucleotide bases.

The term "nucleoside," as used herein, refers to a compound having a nucleotide base covalently linked to the C-1' carbon of a pentose sugar. In some Embodiments, the linkage is via a heteroaromatic ring nitrogen. Typical pentose sugars include, but are not limited to, those pentoses in which one or more of the carbon atoms are each independently substituted with one or more of the same or different -R, -OR, -NRR or halogen groups, where each R is independently hydrogen, (C1-C6) alkyl or (C5-C14) aryl. The pentose sugar may be saturated or unsaturated. Exemplary pentose sugars and analogs thereof include, but are not limited to, ribose, 2'-deoxyribose, 2'-(C1-C6)alkoxyribose, 2'-(C5-C14)aryloxyribose, 2',3'-dideoxyribose, 2',3'-didehydroribose, 2'-deoxy-3'-haloribose, 2'-deoxy-3'-fluororibose, 2'-deoxy-3'-chlororibose, 2'-deoxy-3'-aminoribose, 2'-deoxy-3'-(C1-C6)alkylribose, 2'-deoxy-3'-(C1-C6)alkoxyribose and 2'-deoxy-3'-(C5-C14)aryloxyribose. Also see, e.g., 2'-O-methyl, 4'-$\alpha$-anomeric nucleotides, 1'-$\alpha$-anomeric nucleotides (US Patent No. 4,835,263, Nguyen et al.), 2'-4'- and 3'-4'-linked and other "locked" or "LNA," bicyclic sugar modifications (WO 98/22489; WO 98/39352; WO 99/14226). "LNA" or "locked nucleic acid" is a DNA analogue that is conformationally locked such that the ribose ring is constrained by a methylene linkage between the 2'-oxygen and the 3'- or 4'-carbon.

Sugars include modifications at the 2'- or 3'-position such as methoxy, ethoxy, allyloxy, isopropoxy, butoxy, isobutoxy, methoxyethyl, alkoxy, phenoxy, azido, amino, alkylamino, fluoro, chloro and bromo. Nucleosides and nucleotides include the natural D configurational isomer (D-form), as well as the L configurational isomer (L-form) (Beigelman, U.S. Pat. No. 6,251,666; Chu, U.S. Pat. No. 5,753,789; Shudo, EP0540742). When the nucleobase is purine, e.g., A or G, the ribose sugar is attached to the N9-position of the nucleobase. When the nucleobase is pyrimidine, e.g., C, T or U, the pentose sugar is attached to the N1-position of the nucleobase.

One or more of the pentose carbons of a nucleoside may be substituted with a phosphate ester. In some Embodiments, the phosphate ester is attached to the 3'- or 5'-carbon of the pentose. In some Embodiments, the nucleosides are those in which the nucleotide base is a purine, a 7-deazapurine, a pyrimidine, a universal nucleotide base, a specific nucleotide base, or an analog thereof.

The term "nucleotide analog," as used herein, refers to embodiments in which the pentose sugar and/or the nucleotide base and/or one or more of the phosphate esters of a nucleoside may be replaced with its respective analog. In some embodiments, exemplary pentose sugar analogs are those described above. In some embodiments, the nucleotide analogs have a nucleotide base analog as described above. In some embodiments, exemplary phosphate ester analogs include, but are not limited to, alkylphosphonates, methylphosphonates, phosphoramidates, phosphotriesters, phosphorothioates, phosphorodithioates, phosphoroselenoates, phosphorodiselenoates, phosphoroanilothioates, phosphoroanilidates, phosphoroamidates, boronophosphates, and may include associated counterions. Other nucleic acid analogs and bases include for example intercalating nucleic acids (INAs, as described in Christensen and Pedersen, 2002), and AEGIS bases (Eragen, U.S. Pat. No. 5,432,272). Nucleic acids containing one or more carbocyclic sugars are also included within the definition of nucleic acids

The term "universal nucleotide base" or "universal base," as used herein, refers to an aromatic ring moiety, which may or may not contain nitrogen atoms. In some Embodiments, a universal base may be covalently attached to the C-1' carbon of a pentose sugar to make a universal nucleotide. In some Embodiments, a universal nucleotide base does not hydrogen bond specifically with another nucleotide base. In some Embodiments, a universal nucleotide base hydrogen bonds with nucleotide base, up to and including all nucleotide bases in a particular target polynucleotide. In some Embodiments, a nucleotide base may interact with adjacent nucleotide bases on the same nucleic acid strand by

hydrophobic stacking. Universal nucleotides include, but are not limited to, deoxy-7-azaindole triphosphate (d7AITP), deoxyisocarbostyril triphosphate (dICSTP), deoxypropynylisocarbostyril triphosphate (dPICSTP), deoxymethyl-7-azaindole triphosphate (dM7AITP), deoxylmPy triphosphate (dlmPyTP), deoxyPP triphosphate (dPPTP), or deoxypropynyl-7-azaindole triphosphate (dP7AITP). Further examples of such universal bases can be found, inter alia, in Published U.S. Application No. 10/290672, and U.S. Pat. No. 6,433,134.

As used herein, the terms "polynucleotide" and "oligonucleotide" are used interchangeably and mean single-stranded and double-stranded polymers of nucleotide monomers, including 2'-deoxyribonucleotides (DNA) and ribonucleotides (RNA) linked by internucleotide phosphodiester bond linkages, e.g., 3'-5' and 2'-5', inverted linkages, e.g., 3'-3' and 5'-5', branched structures, or internucleotide analogs. Polynucleotides have associated counter ions, such as H+, NH4+, trialkylammonium, Mg2+, Na+, and the like. A polynucleotide may be composed entirely of deoxyribonucleotides, entirely of ribonucleotides, or chimeric mixtures thereof. Polynucleotides may be comprised of internucleotide, nucleobase and/or sugar analogs. Polynucleotides typically range in size from a few monomeric units, e.g., 3-40 when they are more commonly frequently referred to in the art as oligonucleotides, to several thousands of monomeric nucleotide units. Unless denoted otherwise, whenever a polynucleotide sequence is represented, it will be understood that the nucleotides are in 5' to 3' order from left to right and that "A" denotes deoxyadenosine, "C" denotes deoxycytosine, "G" denotes deoxyguanosine, and "T" denotes thymidine, unless otherwise noted.

As used herein, "nucleobase" means those naturally occurring and those non-naturally occurring heterocyclic moieties commonly known to those who utilize nucleic acid technology or utilize peptide nucleic acid technology to thereby generate polymers that can sequence specifically bind to nucleic acids. Non-limiting examples of suitable nucleobases include: adenine, cytosine, guanine, thymine, uracil, 5-propynyl-uracil, 2-thio-5-propynyl-uracil, 5-methylcytosine, pseudoisocytosine, 2-thiouracil and 2-thiothymine, 2-aminopurine, N9-(2-amino-6-chloropurine), N9-(2,6-diaminopurine), hypoxanthine, N9-(7-deaza-guanine), N9-(7-deaza-8-aza-guanine) and N8-(7-deaza-8-aza-adenine).

As used herein, "nucleobase sequence" means any segment, or aggregate of two or more segments (e.g. the aggregate nucleobase sequence of two or more oligomer blocks), of a polymer that comprises nucleobase-containing subunits. Non-limiting examples of suitable polymers or polymers segments include oligodeoxynucleotides (e.g. DNA), oligoribonucleotides (e.g. RNA), peptide nucleic acids (PNA), PNA chimeras, PNA combination oligomers, nucleic acid analogs and/or nucleic acid mimics.

As used herein, "polynucleobase strand" means a complete single polymer strand comprising nucleobase subunits. For example, a single nucleic acid strand of a double stranded nucleic acid is a polynucleobase strand.

As used herein, "nucleic acid" is a nucleobase sequence-containing polymer, or polymer segment, having a backbone formed from nucleotides, or analogs thereof.

Preferred nucleic acids are DNA and RNA.

As used herein, nucleic acids may also refer to "peptide nucleic acid" or "PNA" means any oligomer or polymer segment (e.g., block oligomer) comprising two or more PNA subunits (residues), but not nucleic acid subunits (or analogs thereof), including, but not limited to, any of the oligomer or polymer segments referred to or claimed as peptide nucleic acids in U.S. Pat. Nos. 5,539,082; 5,527,675; 5,623,049; 5,714,331; 5,718,262; 5,736,336; 5,773,571; 5,766,855; 5,786,461; 5,837,459; 5,891,625; 5,972,610; 5,986,053; and 6,107,470. The term "peptide nucleic acid" or "PNA" shall also apply to any oligomer or polymer segment comprising two or more subunits of those nucleic acid mimics such as those described in Peptide-Based Nucleic Acid Mimics (PENAMS) of Shah et al. as disclosed in WO96/04000.

The lipid particles according to some embodiments of the invention can also be characterized by electron microscopy. The particles of the invention having a substantially solid core have an electron dense core as seen by electron microscopy. One such structure is disclosed in United States Patent No. 9,758,795 by Cullis et al. Electron dense is defined such that area-averaged electron density of the interior 50% of the projected area of a solid core particle (as seen in a 2-D cryo EM image) is not less than x% (x = 20%, 40%, 60%) of the maximum electron density at the periphery of the particle. Electron density is calculated as the absolute value of the difference in image intensity of the region of interest from the background intensity in a region containing no nanoparticle.

The lipid particles of the invention have a diameter (mean particle diameter) from about 15 to about 300 nm. In some embodiments, the mean particle diameter is greater than 300 nm. In some Embodiments, the lipid particle has a diameter of about 300 nm or less, 250 nm or less, 200 nm or less, 150 nm or less, 100 nm or less, or 50 nm or less. In one embodiment, the lipid particle has a diameter from about 50 to about 150 nm. These particles generally exhibit increased circulatory lifetime *in vivo* compared to large particles. In one embodiment, the lipid particle has a diameter from about 15 to about 50 nm.

These particles are capable of advantageously escaping the vascular system. In one embodiment, the lipid particle has a diameter from about 15 to about 20 nm. These particles near the limit size for particles that contain a nucleic acid; such particles may include a single polynucleotide (e.g., siRNA).

The lipid particles of the invention are substantially homogeneous in their size distribution. In certain embodiments, the lipid particles of the invention have a mean particle diameter standard deviation of from about 65 to about 25%. In one embodiment, the lipid particles of the invention have a mean particle diameter standard deviation of about

60, 50, 40, 35, or 30%. In some embodiments, the lipid nucleic acid particles of the invention have a PDI of about 0.01 to 0.3.

The lipid particles according to embodiments of the invention are prepared by a process by which nearly 100% of the nucleic acid used in the formation process is encapsulated in the particles. In one embodiment, the lipid particles are prepared by a process by which from about 90 to about 95% of the nucleic acid used in the formation process is encapsulated in the particles.

Disclosed herein is a method for making lipid particles containing a therapeutic agent.

A variety of methods have been developed to formulate LNP systems containing genetic drugs. These methods include mixing preformed LNP with NAT in the presence of ethanol or mixing lipid dissolved in ethanol with an aqueous media containing NAT and result in LNP with NAT encapsulation efficiencies of 65-95%. Both of these methods rely on the presence of cationic lipid to achieve encapsulation of NAT and a stabilizing agent to inhibit aggregation and the formation of large structures. The properties of the LNP systems produced, including size and NAT encapsulation efficiency, are sensitive to a variety of formulation parameters such as ionic strength, lipid and ethanol concentration, pH, NAT concentration and mixing rates. In general, parameters such as the relative lipid and NAT concentrations at the time of mixing, as well as the mixing rates are difficult to control using current formulation procedures, resulting in variability in the characteristics of NAT produced, both within and between preparations.

Microfluidic devices enable controlled and rapid mixing of fluids on a nanoliter scale. Temperature, residence times, and solute concentrations are controlled during the process of rapid microfluidic mixing applied in the synthesis of inorganic nanoparticles and microparticles, and can outperform macroscale systems in large scale production of nanoparticles. Microfluidic two-phase droplet techniques have been applied to produce monodisperse polymeric microparticles for drug delivery or to produce large vesicles for the encapsulation of cells, proteins, or other biomolecules. The use of hydrodynamic flow focusing, a common microfluidic technique to provide rapid mixing of reagents, to create monodisperse liposomes of controlled size has been demonstrated. This technique has also proven useful in the production of polymeric nanoparticles where smaller, more monodisperse particles were obtained, with higher encapsulation of small molecules as compared to bulk production methods.

U.S. Application Pub. Nos. 20120276209 and 20140328759, by Cullis et al. describe methods of using small volume mixing technology and novel formulations derived thereby. U.S. Application Pub. No. 20160022580 by Ramsay et al. describes more advanced methods of using small volume mixing technology and products to formulate different materials. U.S. Application Pub. No. US2016235688 by Walsh, et al. discloses microfluidic mixers with different paths and wells to elements to be mixed. PCT Publication WO17117647 discloses microfluidic mixers with disposable sterile paths. PCT Publication No WO/2017/11764 by Wild, Leaver and Taylor discloses bifurcating toroidal micromixing geometries and their application to micromixing. US Design patents D771834, D771833 and D772427 by Wild and Weaver disclose cartridges for microfluidic mixers.

Devices for biological microfluidic mixing may be used to prepare the lipid particles and therapeutic formulations of the dislosure and invention. Precision NanoSystems Inc., in Vancouver, Canada, manufactures and sells such devices under the NanoAssemblr™ brand. The devices include a first and second stream of reagents, which feed into the microfluidic mixer, and lipid nanoparticles are collected from the outlet, or in other embodiments, emerge into a sterile environment.

The first stream includes a therapeutic agent in a first solvent. Suitable first solvents include solvents in which the therapeutic agents are soluble and that are miscible with the second solvent. Suitable first solvents include aqueous buffers. Representative first solvents include citrate and acetate buffers.

The second stream includes lipid particle-forming materials in a second solvent. Suitable second solvents include solvents in which the cationic lipids are soluble and that are miscible with the first solvent. Suitable second solvents include 1,4-dioxane, tetrahydrofuran, acetone, acetonitrile, dimethyl sulfoxide, dimethylformamide, acids, and alcohols. Representative second solvents include aqueous ethanol 90%, or anhydrous ethanol.

A suitable device may include one or more microchannels (i.e., a channel having its greatest dimension less than 1 millimeter). In one example, the microchannel has a diameter from about 20 to about 300 $\mu$m. In examples, at least one region of the microchannel has a principal flow direction and one or more surfaces having at least one groove or protrusion defined therein, the groove or protrusion having an orientation that forms an angle with the principal direction (e.g., a staggered herringbone mixer), as described in U.S. Application Publication No. 2004/0262223. In examples, at least one region of the microchannel comprises bas-relief structures. To achieve maximal mixing rates, it is advantageous to avoid undue fluidic resistance prior to the mixing region. Thus, one example of a device has non-microfluidic channels having dimensions greater than 1000 microns, to deliver the fluids to a single mixing channel.

In other examples of mixing technology, the first and second streams are mixed with other micromixers. Suitable micromixers include droplet mixers, T-mixers, zigzag mixers, multilaminate mixers, or other active mixers.

The lipid particles of the present invention may be used to deliver a therapeutic agent to a cell, *in vitro* or *in vivo.* In particular embodiments, the therapeutic agent is a nucleic acid, which is delivered to a cell using nucleic acid-lipid particles of the present invention. The methods and compositions may be readily adapted for the delivery of any suitable therapeutic agent for the treatment of any disease or disorder that would benefit from such treatment.

Methods of in vivo treatment are disclosed herein but do not form part of the claimed invention.

In certain embodiments, the present invention provides methods for introducing a nucleic acid into a cell. Preferred nucleic acids for introduction into cells are siRNA, mRNA, immune-stimulating oligonucleotides, plasmids, antisense and ribozymes. These methods may be carried out by contacting the particles or compositions of the present invention with the cells for a period of time sufficient for intracellular delivery to occur.

Typical applications include using well known procedures to provide intracellular delivery of siRNA to knock down or silence specific cellular targets. Alternatively applications include delivery of DNA or mRNA sequences that code for therapeutically useful polypeptides. In this manner, therapy is provided for genetic diseases by supplying deficient or absent gene products. Methods of the present invention may be practiced *in vitro, ex vivo,* or *in vivo.* For example, the compositions of the present invention can also be used for delivery of nucleic acids to cells *in vivo,* using methods which are known to those of skill in the art.

The delivery of siRNA, mRNA and plasmid nucleic acid therapeutics by a lipid particle of the invention is described below.

For *in vivo* administration, which is disclosed herein but which does not form part of the claimed invention, the pharmaceutical compositions are preferably administered parenterally (e.g., intraarticularly, intravenously, intraperitoneally, subcutaneously, intradermaly, intratrachealy, intraosseous or intramuscularly). In particular embodiments, the pharmaceutical compositions are administered intravenously or intraperitoneally by a bolus injection. Other routes of administration include topical (skin, eyes, mucus membranes), oral, pulmonary, intranasal, sublingual, rectal, and vaginal.

In one embodiment, the present invention provides a method of modulating the expression of a target polynucleotide or polypeptide. These methods generally comprise contacting a cell with a lipid particle of the present invention that is associated with a nucleic acid capable of modulating the expression of a target polynucleotide or polypeptide. As used herein, the term "modulating" refers to altering the expression of a target polynucleotide or polypeptide. Modulating can mean increasing or enhancing, or it can mean decreasing or reducing.

Also described, but not claimed, is a method of treating a disease or disorder characterized by overexpression of a polypeptide in a subject, comprising providing to the subject a pharmaceutical composition of the present invention, wherein the therapeutic agent is selected from an siRNA, a microRNA, an antisense oligonucleotide, and a plasmid capable of expressing an siRNA, a microRNA, or an antisense oligonucleotide, and wherein the siRNA, microRNA, or antisense RNA comprises a polynucleotide that specifically binds to a polynucleotide that encodes the polypeptide, or a complement thereof.

Also described , but not claimed, is a method of treating a disease or disorder characterized by underexpression of a polypeptide in a subject, comprising providing to the subject a pharmaceutical composition of the present invention, wherein the therapeutic agent is selected from an mRNA, a self-replicating DNA, or a plasmid, comprises a nucleic acid therapeutic that specifically encodes or expresses the under-expressed polypeptide, or a complement thereof.

Exemplary mRNA encodes the protein or enzyme selected from human growth hormone, erythropoietin, a 1 -antitrypsin, acid alpha glucosidase, arylsulfatase A, carboxypeptidase N, a-galactosidase A, alpha-L-iduronidase, iduronate-2- sulfatase, iduronate sulfatase, N-acetylglucosamine- 1 -phosphate transferase, N-acetylglucosaminidase, alpha-glucosaminide acetyltransferase, N- acetylglucosamine 6-sulfatase, N-acetylgalactosamine-4-sulfatase, beta- glucosidase, galactose-6-sulfate sulfatase, beta-galactosidase, beta-glucuronidase, glucocerebrosidase, heparan sulfamidase, heparin-N-sulfatase, lysosomal acid lipase, hyaluronidase, galactocerebrosidase, ornithine transcarbamylase (OTC), carbamoyl-phosphate synthetase 1 (CPS 1), argininosuccinate synthetase (ASS 1), argininosuccinate lyase (ASL), arginase 1 (ARGI), cystic fibrosis transmembrane conductance regulator (CFTR), survival motor neuron (SMN), Factor VIII, Factor IX, meganucleases like TALENS, Cas9 and self-replicating RNA's and low density lipoprotein receptors (LDLR).

Also described is a pharmaceutical composition comprising a lipid particle of the invention and a pharmaceutically acceptable carrier or diluent. Representative pharmaceutically acceptable carriers or diluents include solutions for intravenous injection (e.g., saline or dextrose). The composition can take the form of a cream, ointment, gel, suspension, or emulsion.

The following is a description of a representative lipid nucleic acid particle (LNP) system, device and method for making the LNP system, and method for using a LNP for delivering therapeutic agents.

Formulation of LNPs was performed by rapidly mixing a lipid-ethanol solution with an aqueous buffer inside a microfluidic mixer designed to induce chaotic advection and provide a controlled mixing environment at intermediate Reynolds number ($24 < Re < 1000$). The microfluidic channel have herringbone features or configured in a manner as shown in WO/2017/117647.

Particle sizes and "polydispersity index" (PDI) of the LNP were measured by dynamic light scattering (DLS). PDI indicates the width of the particle distribution. This is a parameter calculated from a cumulative analysis of the (DLS)-measured intensity autocorrelation function assuming a single particle size mode and a single exponential fit to the autocorrelation function. From a biophysical point of view, a PDI below 0.1 indicates that the sample is monodisperse. (As a reference, PDI of the NIST standards are below 0.05.) The particles produced are homogeneous in size.

The following examples and embodiments are provided for the purpose of illustrating, not limiting, the claimed invention.

A stabilizing agent may be present in the particle in an amount from about 0.1 to about 20MOL%. In some examples, the stabilizing agent is a surfactant. In some examples, the stabilizing agent is a PEG Lipid.

The stabilizing agent may be present in the particle in an amount from about 0.5 to about 10MOL%. The stabilizing agent may be present in the lipid nanoparticle at about 2MOL%. According to the invention, the stabilizing agent is present at 2.5MOL%.

Preferably, the stabilizing agent is polyoxyethylene (40) stearate. In another example, the stabilizing agent is polyoxyethylene (20) oleyl ether. In yet another example, the stabilizing agent is polyoxyethylene (44) stearate. "Myrj52™" is a tradename for polyoxyethylene (40) stearate. It is sold by Sigma-Aldrich Canada Co.

In certain examples, the stabilizing agent is a polyethylene glycol-lipid. Suitable polyethylene glycol-lipids include PEG-modified phosphatidylethanolamine, PEG-modified phosphatidic acid, PEG-modified ceramides (e.g., PEG-CerC14 or PEG-CerC20), PEG-modified dialkylamines, PEG-modified diacylglycerols, PEG-modified dialkylglycerols. Representative polyethylene glycol-lipids include PEG-c-DOMG, PEG-c-DMA, and PEG-s-DMG. Many of these are for sale from NOF America Corporation, White Plains, NY under brand names such as SUNBRIGHT® GM-020(DMG-PEG)

The stabilizing agent can be a non-ionic surfactant. In some examples, the Stabilizing agent is a PEG-lipid is present at concentrations of 0 to 10MOL%. The stabilizing agent can be vitamin E TPGS. In some examples, the repeating PEG moiety has an average molecular weight of 1000 -2000. In some examples, molecular weight average of the PEG segment is 1700 - 2000 with n value (number of PEG repeating units) between 39 - 47.

In other preferred embodiments, the nucleic acid is a plasmid composed of double stranded deoxyribonucleic acid. A plasmid is a genetic structure that resides in a cell's cytoplasm (as opposed to the nucleic where the traditional cellular genetics reside) cell that can replicate independently of the chromosomes, typically a small circular DNA strand. This is not a normal mammalian genetic construct, but is used as a therapeutic option for replacing or restoring faulty genetic function in a cell. Plasmids can also be used to create novel cellular or animal models for medical research. An engineered plasmid will have, in addition to a replication origin (or not, depending on the intended use), restriction enzyme recognition sites, which allow breaking the circle to introduce new genetic material, and a selective marker such as an antibiotic resistance gene. A plasmid may be from 2,000 to about 1 million base pairs (bp). The larger the plasmid, the more susceptible it is to shearing forces.

Further definitions.

DiD means 'DiD'; DilC18(5) oil (1,1'-Dioctadecyl-3,3,3',3'-Tetramethylindodicarbocyanine Perchlorate) (Invitrogen D-307) and is a lipid label. It is useful for laboratory work but has not been tested for human use. It is not an intended component of the final product. Other such agents are dansyl DOPE and Fluorescein DHPE. These are non transferable, remaining with the lipid nanoparticles to indicate their location.

As used herein, the term "about" is defined as meaning 10% plus or minus the recited number. It is used to signify that the desired target concentration might be, for example, 40 MOL %, but that through mixing inconsistencies, the actual percentage might differ by +/- 5 MOL %.

As used herein, the term "nucleic acid" is defined as a substance intended to have a direct effect in the diagnosis, cure, mitigation, treatment or prevention of disease, or to have direct effect in restoring, correcting or modifying physiological functions, or to act as a research reagent. In preferred embodiments, the nucleic acid is an oligonucleotide. In preferred embodiments, the therapeutic agent is a nucleic acid therapeutic, such as an RNA polynucleotide. In preferred embodiments, the therapeutic agent is double stranded circular DNA (plasmid).

As used herein, the term "research reagent" is defined by the fact that it has a direct influence on the biological effect of cells, tissues or organs. Research reagents include but are not limited polynucleotides, proteins, peptides, polysaccharides, inorganic ions and radionuclides. Examples of nucleic acid research reagents include but are not limited to antisense oligonucleotides, ribozymes, microRNA, mRNA, ribozyme, tRNA, tracrRNA, sgRNA, snRNA, siRNA, shRNA, ncRNA, miRNA, mRNA, pre-condensed DNA, pDNA or an aptamer. Nucleic acid Research Reagents are used to silence genes (with for example siRNA), express genes (with for example mRNA), edit genomes (with for example CRISPR/Cas9).

In this disclosure, the word "comprising" is used in a non-limiting sense to mean that items following the word are included, but items not specifically mentioned are not excluded. It will be understood that in embodiments which comprise or may comprise a specified feature or variable or parameter, alternative embodiments may consist, or consist essentially of such features, or variables or parameters. A reference to an element by the indefinite article "a" does not exclude the possibility that more than one of the elements is present, unless the context clearly requires that there be one and only one of the elements.

In this disclosure, "transfecting reagent" means a composition that enhances the transfer of nucleic acid into cells. It typically includes a cationic lipid to associate with nucleic acid, and structural lipids. LIPOFECTIN™ and LIPOFECTAMINE™ are legacy transfecting reagent. MESSENGER MAX™ LIPOFECTAMINE™ is a contemporary transfecting reagent.

In this disclosure the recitation of numerical ranges by endpoints includes all numbers subsumed within that range including all whole numbers, all integers and all fractional intermediates (e.g., 1 to 5 includes 1, 1.5, 2, 2.75, 3, 3.80, 4, and 5 etc.). In this disclosure the singular forms an "an", and "the" include plural referents unless the content clearly dictates otherwise. Thus, for example, reference to a composition containing "a compound" includes a mixture of two or more compounds.

In this disclosure term "or" is generally employed in its sense including "and/or" unless the content clearly dictates otherwise.

The following examples are provided for the purpose of illustrating, not limiting, the disclosure and invention.

Materials

1,17-bis(2-octylcyclopropyl)heptadecan-9-yl-4-(dimethylamino)butanoate hydrochloride 1,2-dioleoyl-sn-glycero-3-phosphocholine (DOPC), 1,2-distearoyl-sn-glycero-3-phosphocholine (DSPC)Ammonium acetate, sodium acetate and sodium chloride were obtained from Fisher Scientific (Fair Lawn, NJ). RNase A was obtained from Applied Biosystems/Ambion (Austin, TX), 1,17-bis(2-octylcyclopropyl)heptadecan-9-yl 4-(dimethylamino) butanoate was synthesized by Avanti Polar Lipids (Alabaster, AL, USA), and PEG (40) stearate was from Sigma St Louis, MO, USA.

Methods

Oligonucleotide or polynucleotide (siRNA, mRNA or plasmid, hereinafter referred to as "nucleic acid") solution was prepared in 25 mM-100 mM acetate buffer at pH 4.0. Depending on the desired oligonucleotide-to-lipid ratio and formulation concentration, solutions were prepared at a target concentration of 2.3 mg/ml to 4 mg/ml total lipid. A lipid solution containing 1,17-bis(2-octylcyclopropyl)heptadecan-9-yl 4-(dimethylamino)butanoate hydrochloride, a selection of one of DSPC, DOPC, DOPE, Myrj52 at 1 or 2.5MOL% or PEG-lipid at 0-10%, DiD label was prepared in ethanol and mixed with the oligonucleotide or polynucleotide to achieve an ethanol concentration of 25% (v/v). Optionally, cholesterol, was present at about 20-40%. Later tests showed that cholesterol presence had no impact on cell toxicity or transfection.

LNP were prepared by standard processes described above. Mixing occurred by chaotic advection, causing the separation of laminate streams to become increasingly small, thereby promoting rapid diffusion. This mixing occurs on a millisecond time scale and results in the lipids being transferred to a progressively more aqueous environment, reducing their solubility and resulting in the spontaneous formation of lipid nanoparticles (LNP). By including cationic lipids in the lipid composition, entrapment of oligonucleotide or polynucleotide species is obtained through association of the positively charged lipid head group and negatively charged oligonucleotide.

Following mixing in the microfluidic device, the LNP mixture was generally diluted into RNAse free tubes containing three to forty volumes of stirred phosphate buffered saline (PBS) buffer, pH 7.4. Ethanol is finally removed through dialysis in PBS, pH 7 or using amicon centrifugal filters (Millipore, USA) at 3000 RPM. Once the required concentration is achieved, the particles were filter sterilized using 200 um filters in aseptic conditions. Empty vesicles were similarly produced, with the oligonucleotide absent from the buffer solution.

Particle size was determined by dynamic light scattering using a ZetaSizer Nano ZS™, Malvern Instruments, UK). Lipid concentrations were verified by measuring total cholesterol using the Cholesterol E enzymatic assay from Wako Chemicals USA (Richmond, VA).

RNA concentration and Encapsulation Efficiency was measured by a modified RiboGreen assay (Quant-IT-Ribogreen® Assay kit, Invitrogen). Encapsulation efficiency is defined as the percentage of RNA protected with in the LNP. Briefly, Standards and samples were prepared in a 96 well plate with and without 1% Triton- X100. 96 well plate was incubated at 37 ▯C for 15 minutes to break open the RNA LNPs. On completion of incubation, Ribogreen® reagent was added to samples and standards, and fluorescence intensities of standards and samples with and without triton were measured (Exc.485nm/Em 520nm). Percent Encapsulation or Encapsulation Efficiency is defined by the following equation.

$$\textit{Encapsulation Efficiency} = [(\textit{Total RNA} - \textit{Free RNA (un-encapsulated RNA)}) \square \textit{ Total RNA}]\%$$

For the culture of neurons, microsurgically dissected Cortex tissue from E18 Sprague Dawley rat was purchased from BrainBits, LLC, Springfield, IL. This tissue was processed and the neuronal cells plated using the neuronal plating protocol from StemCell Technologies, Vancouver, BC. In short the E18 cortex tissues were removed from the shipping medium and digested for 20 minutes using 0.25% trypsin. Following this the trypsin was inactivated using DMEM media containing 10% FBS. The tissue was then pelleted and the FBS containing media was replaced with fresh DMEM. Following this the tissue was again pelleted and the pellet was triturated in Neuronal Culture Media supplemented with

SM1, (StemCell Technologies). The cell suspension was then passed through a 40μM cell strainer_to form a single cell suspension. The concentration of the cell suspension was assessed using trypan blue and a hemocytometer. The cell suspension was seeded at a density of $4.8 \times 10^4$ cells/cm$^2$ on PDL coated plates or at a density of $3.2 \times 10^4$ cells/cm$^2$ on PDL coated coverslips. The cells were incubated in a 37°C incubator with 5% $CO_2$ and half of the media was changed with fresh media every 3-4 days. Confirmation of neuronal cell type was done using visual inspection of the culture by microscope as well as positive MAP2 staining, a marker of neuronal cells.

For the culture of astrocytes, microsurgically dissected Cortex tissue from E18 Sprague Dawley rat was purchased from BrainBits, LLC, Springfield, IL. This tissue was then processed by digestion with 0.25% trypsin for 20 minutes followed by inactivation of the trypsin by the addition of astrocyte medium (DMEM media containing 10% FBS and 2mM of L-gluatmine). Following centrifugation the tissue pellet was resuspended in astrocyte medium, triturated to form a cell suspension, and passed through a 40μM cell strainer to form a single cell suspension. The concentration of the cell suspension was assessed using Trypan blue and a hemocytometer. The suspension was then seeded at a density of $1.33 \times 10^4$ cells/cm$^2$ in PDL coated T150 flasks. The cells were incubated in a 37°C incubator with 5% $CO_2$ following seeding and the media was changed every 3-4 days. After 10 days in culture the flasks were fully confluent and the astrocyte morphology of the cells could clearly be seen. The astrocytes were harvested by tapping the flasks to remove any remaining microglia in the culture and then by washing the flask with PBS without calcium or magnesium. 0.25% trypsin was added to detach the astrocytes from the flask through incubation for 5 minutes at 37°C. DMEM media containing 10% FBS and 2mM of L-glutamine was then added to inactivate the trypsin. A cell scraper was used to ensure that all the astrocytes were detached from the flask as well as a titration and washing of the flask with the media. The resulting cell suspension was then counted using Trypan blue and a hemocytometer and the astrocytes were then either plated directly for experiments or cryopreserved for later expansion and use. For experiments, the astrocytes were seeded on tissue culture plates at a density of $2 \times 10^4$ cells/cm$^2$ in regular tissue culture treated plates or on Poly-D-lysine (PDL) coated coverslips. Confirmation of astrocyte cell type was done using visual inspection of the culture by microscope as well as positive GFAP staining, a marker of astrocyte cells. Human iPSC-Derived Neural Progenitor Cells. Human neural progenitor cells derived from male human pluripotent stem cell line XCL-1, Size: $1 \times 10^6$ cells, purchased from StemCell Technologies, Vancouver, Canada. They were cultured in neural progenitor medium 2, also purchased from StemCell, according to vendor directions.

Cell treatment

Neurons: After 7 days in culture, neurons were treated with 2.5μg/mL of mRNA LNP and supplemented with 5μg/mL of ApoE (neurons do not produce enough ApoE in enriched cultures to facilitate uptake of the LNP). 48H after treatment, the neurons were harvested for downstream assessment. Best results were obtained when both mRNA and plasmid were added to human NPCs when they were seeded. Thus when the cells are passaged,they were seeded in individual wells and then the APoE and LNP added immediately.

Astrocytes: 48H after being seeded, the astrocytes were treated with 2.5μg/mL of mRNA LNP. 24H after treatment, the astrocytes were harvested for downstream assessment.

Human iPSC-Derived Neural Progenitor Cells ("NPC")

NPC were treated with 100 ng/mL of mRNA or plasmid at the time of seeding, with 1 ug/ml ApoE. Samples were then assayed using flow cytometry at 48 h post seeding.

Immunocytochemistry

For either neurons or astrocytes, cells were seeded at the appropriate density on PDL coated coverslips. Following treatment and incubation, the cells were fixed by 4% paraformaldehyde. The cells were permeabilized using 0.1% Triton X-100 in PBS and then blocked using 10% normal donkey serum. Following blocking, the primary antibody was added, MAP2 for neuronal cells and GFAP for astrocytes, and incubated overnight at 4°C. The following day, the primary antibody was removed and secondary antibody was added as well as DAPI for staining the nucleus. The coverslips were then mounted on glass slides using ProLong Diamond™ fixative, ThermoScientific. Images were acquired using a confocal microscope.

Viability assay

Neurons or astrocytes cells were seeded at the appropriate density in 96 well plates in a final media volume per well of 100μl. Following treatment and treatment incubation time 10μl of medium was removed and 10μl of Presto-Blue® cell viability reagent, ThermoScientific, was added and the plate was incubated for_30 minutes at 37°C before

being read using a plate reader (Synergy H1 plate reader (BioTek) using the reader specifications in the PrestoBlue® cell viability reagent.

Flow Cytometry

Following treatment and treatment incubation of either neurons or astrocytes the media from each well was collected and the cells were harvested using 0.25% trypsin. The trypsin was inactivated using 3% FBS in PBS and the cells were pelleted in their corresponding media. Following centrifugation the cells were washed once with PBS and again pelleted. The cells were then resuspended in Binding Buffer, BD Biosciences, and Propidium Iodine was added to stain for cells with completely ruptured membranes, dead cells. The cells were then assessed using a BD Biosciences Canto II flow cytometer.

The comparison of Lipid Mix A and Lipid Mix D flow cytometry results for mRNA are shown in Fig. 6. Lipid Mix D is far superior.

GFP ELISA

Following treatment and treatment incubation of either neurons or astrocytes the cells were harvested following the protocol provided with SimpleStep™ GFP ELISA, AbCam. In short the cells were washed with PBS and then lysed using the provided lysis buffer. Following lysis and collection the lysate was incubated on ice and then centrifuged to remove any remaining undigested cell debris. Total protein concentration was assessed using the BCA Protein Quantification Kit (AbCam, Cambridge, UK). The total protein concentration for each sample was then normalized to 1ng/μl for use in the SimpleStep™ GFP ELISA.

Example 1

LNP Characterization

Particle size (hydrodynamic diameter of the particles) was determined by Dynamic Light Scattering (DLS) using a ZetaSizer Nano ZS™, Malvern Instruments, UK). He/Ne laser of 633 nm wavelength was used as the light source. Data were measured from the scattered intensity data conducted in backscattering detection mode (measurement angle 173°). Measurements were an average of 10 runs of two cycles each per sample. Z -Average size was reported as the LNP size, and is defined as the harmonic intensity averaged particle diameter.

RNA encapsulated was measured using the Ribogreen® dye method. The RiboGreen® dye is a fluorescent nucleic acid stain for quantitating intact RNA. RiboGreen® assay provides RNA quantitation with minimal consumption of sample. A standard curve established using known concentrations of control RNA sample. Then the test solutions are run and their measurements translated using the standard curve.

Table 1. Physico-chemical parameters of GFP mRNA LNP Particles

| Formulation ID | Encap. Efficiency (%) | Size (nm) | PDI |
|---|---|---|---|
| Lipid Mix A | 98 | 87 | 0.12 |
| Lipid Mix B | 89 | 113 | 0.15 |
| Lipid Mix C | 98 | 122 | 0.10 |
| Lipid Mix D | 98 | 127 | 0.18 |
| Lipid Mix E | 96 | 133 | 0.19 |
| Lipid Mix F | 90 | 140 | 0.16 |

Formulations tested.

Formulations contained various ratios of the cationic lipid 1,17-bis(2-octylcyclopropyl)heptadecan-9-yl 4-(dimethylamino)butanoate hydrochloride, structural lipids selected from DSPC and DOPE, Myrj52 as a stabilizing agent, and 0.5MOL% of DiD. In some formulations, cholesterol was present. All of the formulations shown in Table 1 had good encapsulation efficiency, size and PDI characteristics.

A list of components ratios for the Lipid Mixes is shown in Table 2. Lipid mix A-C, E, F are outside of the scope of the claims. Lipid mix D is according to the invention.

Table 2: Components and Ratios of Lipid Mix A - F

| | |
|---|---|
| Lipid mix A | 50 MOL% cationic lipid /10MOL % DSPC/ 37.5 MOL % cholesterol/2.5 MOL % Myrj52 |
| Lipid mix B | 50 MOL% cationic lipid/20 MOL% DOPE/27.5 MOL % cholesterol/2.5 MOL % Myrj52 |
| Lipid mix C | 40 MOL% cationic lipid /20 MOL % DOPE/37.5 MOL % cholesterol /2.5 MOL % Myrj52 |
| Lipid mix D | 40 MOL% cationic lipid /40 MOL % /DOPE/17.5 MOL % cholesterol/2.5 Myj52 |
| Lipid mix E | 50 MOL% cationic lipid/30 MOL % DOPE/17.5 MOL % cholesterol/ 2.5 MOL % Myrj52 |
| Lipid mix F | 60 MOL% cationic lipid /20 MOL% DOPE/17.5 MOL% cholesterol/ 2.5 MOL % Myrj52 |

Example 2

mRNA Transfection and Expression in Neurons

Messenger RNAs reagents were purchased from Trilink Biotechnologies, San Diego, CA. The mRNA was used to study the transfection ability of the nanoparticles.

EGFP mRNA (5meC, Ψ) Enhanced Green Fluorescent Protein mRNA (5-methylcytidine, pseudouridine), 1.0 mg/mL in 10 mM Tris-HCl, pH 7.5, Length: 996 nucleotides (Seq Id. No 1). Then unknown bases are a portion of alpha globin which the vendor would not reveal.

Encapsulation efficiency was established for formulations as set out *supra* in Table1. Size of the nanoparticles, as well as homogeneity of the size of the nanoparticles (PDI) was measured.

LNP Formulations Lipid Mix A, B, C, and D containing messenger RNA (GFP) were tested on enriched neurons in order to identify those that are most promising for GFP expression. A table of the observed intensity of signals in photographs, ELISAS, and flow cytometry as shown in the columns below was prepared to aid comparing the Lipid Mixes. "Not interpreted" means that observations were not recorded in this manner.

Table 3: Qualitative Results for Different Formulations - mRNA

| Cell Types | **Neurons** | **Neurons** | **Neurons** | **Neurons** | **Astroctyes** | **Astrocytes or Neurons** | **MRNA** | **siRNA** |
|---|---|---|---|---|---|---|---|---|
| Readouts | Immuno | ELISA (GFP expression) | Flow cytometry - Mean Fluorescen ce Intensity | Flow cytometry- % of Neurons expressing GFP | ELISA (GFP expression) | Preference | Overall | Overall |
| Formulatio n ID | Individual scores | | | | | | | |
| **Lipid Mix A** | - | + | + | + | Not interpreted | A | Poor or Low | Good |
| **Lipid Mix B** | - | Not done | + | + | Not interpreted | A | Poor | Good |
| **Lipid Mix C** | + | Not done | ++ | ++ | Not interpreted | N | Med | Good |
| **Lipid Mix D** | ++ | ++ | +++ | +++ | +++ | Both | Good | Good |
| **Lipid Mix E** | Not interpret ed | Not interpreted | + | + | Not done | Not done | Low | Good |
| **Lipid Mix F** | Not interpret ed | Not interpreted | + | | Not done | Not done | Low | Good |

The formulations contained structural lipids in the form of DSPC or DOPE at ratios ranging from 10 to 40MOL%. Cationic lipid was present in the ratio of 35 to 50MOL%. Cholesterol was present at 17 or 37 or 38.5MOL%. Stabilizing agent was present from 1 or 2.5MOL%. DiD was present in all of the formulations at 0.5MOL%, as it was a necessary label.

The goal was to transfect and have expressed mRNA in neurons. Lipid Mix A and Lipid Mix B gave poor expression of mRNA. Lipid Mix A had provided good transfection for siRNA in an earlier baseline experiment, so it was surprising that it did not work for mRNA. Lipid Mix A contained 50/10/37/2.5 cationic lipid/Structural Lipid/ Cholesterol/ Stabilizing agent, with structural lipid being DSPC in Lipid Mix A.

Figures 1 through 3 are photographic images rendered into grayscale for the purposes of the present application. All images include DAPi, a nuclear stain. Figure 1 is a photograph of live neurons that have been treated with labelled Lipid Mix D formulated nanoparticles containing GFP mRNA. The top right quadrant shows MAP2 (a neuronal marker) antibody staining, the bottom right is DiD lipid labelling of the nanoparticle; the bottom left is GFP expression in the neurons, and the top left quadrant is a merged image showing DiD, MAP2, and GFP, establishing GFP expression in the live neurons. Figure 2 shows staining perspectives to illustrate the ability of Lipid Mix B organized as for Figure 1. Figure 3 is a photograph showing the same staining perspectives for Lipid Mix C.

Lipid Mix A and Lipid mix D mRNA nanoparticles were compared to the same mRNA in MessengerMax™ transfection reagent. In Figure 5, the translated mRNA is visualized by mean fluorescence intensity of GFP expression levels in neurons for the three test nanoparticles.

ELISA results are shown comparing Lipid Mix A and Lipid Mix D formulations for GFP mRNA are shown in Figure 4, which is a bar graph showing the translated mRNA expression levels in neurons by ELISA for Lipid Mix A and Lipid Mix D nanoparticles. The Lipid Mix D efficiency of transfection is much better than that of Lipid Mix A, B, C, with not all results shown.

Figure 10 is a bar graph showing Mean Fluorescence Intensity of GFP expression in human neural progenitor cells treated with plasmid LNP formulations of Lipid Mix A, Lipid Mix B, Lipid Mix C, Lipid Mix D , Lipid Mix E and Lipid Mix F by flow cytometry.

This data suggests that by lowering the cationic lipid composition to 40MOL%, surprising activity can be achieved [Lipid Mix A, Lipid Mix B and Lipid Mix E contained 50MOL% cationic lipid, Lipid Mix F contained 60MOL% cationic lipid and Lipid Mix C and Lipid Mix D contained 40MOL% cationic lipid.] All Formulations had stabilizing agent present at 2.5%. The mole ratios of structural lipids were adjusted such that total MOL% was 100.

Human neural progenitor cells were used as model for mRNA expression using Lipid Mix D mRNA LNP. A flow cytometry histogram showing showing mean fluorescence intensity of GFP expression in human neural progenitor cells (grey histogram) compared to untreated cells (black histogram) is shown in Figure 7. Percentage (%) of cells expressing GFP and Mean Fluorescent Intensity (MFI) levels via flow cytometry for neural progenitor cells treated with Lipid Mix A and Lipid Mix D plasmid LNPs 48 hours post exposure is shown in the bar graph of Figure 9.

Lipid Mix C LNPs showed moderate levels of activity and preferential expression in neurons compared to Lipid Mix B, whereas Lipid Mix B showed better levels of expression in astrocytes than in neurons

Example 3

siRNA Transfection Procedures

mRNA silencing by RNA interference is induced by double stranded oligoribonucleotides with a length of about 22 base pairs with 2-nucleotide long 3' overhangs. This type of small dsRNA is called "small interfering RNA" or "siRNA". For experimental purposes, siRNA can be produced synthetically and then transfected into the target cells, or it can be expressed *in vitro* or *in vivo* using a specially designed expression plasmid.

Enriched neurons were treated with Lipid Nanoparticles containing an off-the-shelf dicer-substrate siRNA (DsiRNA) HPRT (SEQ ID NO 2, sense, SEQ ID NO 3, antisense)) or control siRNA DS NC1, a nonsilencing, negative control DsiRNA that does not recognize any sequences in human, mouse, or rat transcriptomes (SEQ ID NO 4, sense, SEQ ID NO 5, antisense). Both DsiRNA and DsNC1 were purchased from Integrated DNA Technologies, Inc., Coralville, Iowa, U.S.)

HPRT is an acronym for hypoxanthine phosphoribosyltransferase 1, a transferase that catalyzes conversion of hypoxanthine to inosine monophosphate and guanine to guanosine monophosphate. Uptake of HPRT siRNA was measured of Day 0, Day 2, Day 14 and Day 28. Knockdown of HPRT by said siRNA was measured at the same timepoints. Cell viability across LNP concentrations (0 - 5000 ng/ml) showed no toxicity at these concentrations. Stability data of siRNA LNP generated up to 28 days indicated that the siRNA LNP was stable and that there was no change in its activity/potency over this period. SiRNA LNP (1000, 100 and 10 ng/ml) along with a non-coding control siRNA formulation was used as a control when collecting the gene knockdown data.

Cell lysates were collected and the HPRT mRNA levels were measured by qPCR. Data was normalized against the house keeping gene $\beta$-actin. All tested formulations were capable of delivering siRNA successfully to the neurons.

Data is not shown.

Example 4

Plasmid Transfection

Plasmid preparation pCX-EGFP Plasmid size 5514 nt, SEQ ID NO 6, custom made by GenScript USA Inc, Piscataway, NJ, including ampicillin resistance, restriction enzyme HINDIII, in $ddH_2O$. The plasmid included a GFP expressing component which produces target protein only when the plasmid is expressed within a cell.

Lipid Mix A and Lipid Mix D with plasmid encapsulated was added to cell cultures of neurons and astrocytes as described above. Excellent expression was noted in the cell culture treated with Lipid Mix D encapsulated plasmid.

The results of the plasmid encapsulation for Lipid Mix A, and Lipid Mix D are shown in Table 4. There was good encapsulation in the two formulations shown in Table 3, with excellent polydispersity.

Table 4: Plasmid LNP Particles : Physico-chemical parameters

| Formulation ID | Encapsulati on Efficiency (%) | Size | PDI |
|---|---|---|---|
| **Lipid Mix A** | 99 | 94 | 0.223 |
| **Lipid Mix D** | 99 | 88 | 0.149 |

Plasmid LNPs comprised of Lipid Mix B (50 MOL% cationic lipid) and Lipid Mix C (40 MOL% cationic lipid) were tested in neural progentor cells. Human neural progenitor cells derived from male human pluripotent stem cell line XCL-1, Size: 1 × 106 cells more purchased from StemCell Technologies, Vancouver, Canada. They were cultured in Neural Progenitor Medium 2 also purchased from StemCell. Flow cytometry results are shown in Figure 8, and were measured for Mean Fluorescent Intensity (MFI) levels (bar graph on the left), and Percentage (%) of cells expressing GFP (bar graph on the right).

Example 5

Improvement over Commercial Standard

The viability of astrocytes 48h post treatment with the mRNA LNP formulation Lipid Mix D and a commercial formulation MessengerMax™ Lipofectamine™ transfecting agent (ThermoFisher Scientific, USA, web order) as measured by the intensity of the cells on treatment with PrestoBlue® is shown in Figure 6. Lipofectamine™ MessengerMAX™ Transfection Reagent is a transfection reagent marketed for the delivery of mRNA, small RNA, and short dsDNA or HDR templates. PrestoBlue® is a cell viability indicator.

The provided example shows that MessengerMax™ is somewhat toxic in this setting to astrocytes at the concentrations of 1000 - 2500 ng/mL. Similar results were seen in neurons treated with MessengerMax™ and Lipid Mix D. Lipid Mix D lipid nanoparticles did not show any signs of toxicity below doses of 5 ug/mL. These results are not shown.

While specific embodiments of the invention have been described and illustrated, such embodiments should be considered illustrative of the invention only and not as limiting the invention as construed in accordance with the accompanying claims.

Bibliography

Akhtar, S., E. Basu S Fau - Wickstrom, R. L. Wickstrom E Fau - Juliano and R. L. Juliano (1991). "Interactions of antisense DNA oligonucleotide analogs with phospholipid membranes (liposomes)." (0305-1048 (Print)).

Kauffman, K. J., M. J. Webber and D. G. Anderson (2015). "Materials for non-viral intracellular delivery of messenger RNA therapeutics." J Control Release.

Kaufmann k, Dorkin Robert J; Yang, Jung H; Heartlein, Michael W; De Rosa, Frank; Mir, Faryal F; Fenton, Owen S; Anderson, Daniel G (2015). "Optimization of Lipid Nanoparticle Formulations for mRNA Delivery in Vivo with Fractional Factorial and Definitive Screening Designs." Nano Letters 15: 7300-7306.

Lv, H., S. Zhang, B. Wang, S. Cui and J. Yan (2006). "Toxicity of cationic lipids and cationic polymers in gene delivery." Journal of Controlled Release 114(1): 100-109.

Mingozzi, F. and K. A. High (2013). "Immune responses to AAV vectors: overcoming barriers to successful gene

therapy." Blood 122(1): 23-36.
O'Mahony, A. M., B. M. Godinho, J. F. Cryan and C. M. O'Driscoll (2013). "Non-viral nanosystems for gene and small interfering RNA delivery to the central nervous system: formulating the solution." J Pharm Sci 102(10): 3469-3484.
Tam, Y. Y., S. Chen and P. R. Cullis (2013). "Advances in Lipid Nanoparticles for siRNA Delivery." Pharmaceutics 5(3): 498-507.

## Claims

1. A transfection reagent composition comprising:

(a) 40 MOL% of 1,17-bis(2-octylcyclopropyl)heptadecan-9-yl-4-(dimethylamino)butanoate, or a pharmaceutically acceptable salt thereof;

(b) 10-40 MOL% 1,2-dioleoyl-sn-glycero-3-phosphoethanolamine (DOPE);
(c) 10-20 MOL% cholesterol; and
(d) 2.5MOL% stabilizing agent.

**2.** The composition of any one of Claim 1, wherein the stabilizing agent is selected from the group consisting of polyoxyethylene (20) oleyl ether, polyoxyethylene (23) lauryl ether, polyoxyethylene (40) stearate, and PEG-conjugated lipid.

**3.** The composition of claim 2 wherein the stabilizing agent is 1,2-Dimyristoyl-sn-glycerol, methoxypolyethylene glycol (PEG-DMG).

**4.** The composition of any one of Claims 1-3, further comprising a nucleic acid.

**5.** The composition of Claim 4, wherein the nucleic acid is a DNA, an RNA, a locked nucleic acid, a nucleic acid analog, or a plasmid capable of expressing an RNA.

**6.** The composition of Claim 4, wherein the nucleic acid is an antisense oligonucleotide, ribozyme, miRNA, rRNA, tRNA, siRNA, saRNA, snRNA, snoRNA, IncRNA, piRNA, tsRNA, srRNA, crRNA, tracrRNA, sgRNA, shRNA, ncRNA, mRNA, pre-condensed DNA, pDNA, an aptamer, or a combination thereof.

**7.** The composition of any one of claims 1-6, wherein a target cell is selected from the group consisting of a neuron, an astrocyte, or a progenitor cell.

**8.** The composition of any one of Claims 1-7, wherein the composition exists in the

form of nanoparticles having a diameter of from about 15nm to about 300nm, as measured by dynamic light scattering (DLS),
wherein the term "about" means 10% plus or minus the recited number.

**9.** The composition of any one of claims 1-8 wherein the composition exists in the

form of nanoparticles having a diameter of from about 80nm to 150nm, as measured by dynamic light scattering (DLS), wherein
the term "about" means 10% plus or minus the recited number.

**10.** The composition of any of claims 1 to 9 comprising:

(a) 40 MOL% of 1,17-bis(2-octylcyclopropyl)heptadecan-9-yl-4-(dimethylamino)butanoate, or a pharmaceutically acceptable salt thereof;
(b) 40 MOL% 1,2-dioleoyl-sn-glycero-3-phosphoethanolamine (DOPE);
(c) 17.5 MOL% cholesterol; and
(d) 2.5MOL% polyoxyethylene (40) stearate.

**11.** An in vitro method for introducing a nucleic acid into a cell, while maintaining activity of the nucleic acid and viability of the cell, comprising contacting the cell with the composition of any one of Claims 4-10.

**12.** An in vitro method for modulating the expression of a target polynucleotide or polypeptide in a cell, while maintaining cell viability, comprising contacting a cell with the composition of any one of Claims 4-10, wherein the nucleic acid is capable of modulating the expression of a target polynucleotide or polypeptide.

**13.** The method of claim 11 or claim 12, wherein the cell is selected from a group consisting of a mammalian neuron, an astrocyte, or a progenitor cell.

**14.** A method of manufacturing a lipid nanoparticle comprising the composition of any one of claims 1-10 capable of targeting neurons, the method including increasing the ratio of cationic lipid to structural lipid in the lipid nanoparticle.

**15.** A method of manufacturing a lipid nanoparticle comprising the composition of any one of claims 1-10 capable of targeting astrocytes, the method including decreasing the ratio of cationic lipid to structural lipid in the lipid nanoparticle.

## Patentansprüche

1. Transfektionsreagenszusammmensetzung, umfassend:

   (a) 40 MOL% 1,17-Bis(2-octylcyclopropyl)heptadecan-9-yl-4-(dimethylamino)butanoat oder ein pharmazeutisch unbedenkliches Salz davon;
   (b) 10-40 MOL% 1,2-Dioleoyl-sn-glycero-3-phosphoethanolamin (DOPE);
   (c) 10-20 MOL% Cholesterin; und
   (d) 2,5 MOL% Stabilisierungsmittel.

2. Zusammensetzung nach einem von Anspruch 1, wobei das Stabilisierungsmittel ausgewählt ist aus der Gruppe bestehend aus Polyoxyethylen(20)-oleylether, Polyoxyethylen(23)-laurylether, Polyoxyethylen(40)-stearat und PEG-konjugiertem Lipid.

3. Zusammensetzung nach Anspruch 2, wobei es sich bei dem Stabilisierungsmittel um 1,2-Dimyristoyl-snglycerin, Methoxypolyethylenglykol (PEG-DMG) handelt.

4. Zusammensetzung nach einem der Ansprüche 1-3, ferner umfassend eine Nukleinsäure.

5. Zusammensetzung nach Anspruch 4, wobei es sich bei der Nukleinsäure um eine DNA, eine RNA, eine *locked*-Nukleinsäure, ein Nukleinsäureanalog oder ein Plasmid mit der Fähigkeit zur Expression einer RNA handelt.

6. Zusammensetzung nach Anspruch 4, wobei es sich bei der Nukleinsäure um ein Antisense-Oligonukleotid, Ribozym, miRNA, rRNA, tRNA, siRNA, saRNA, snRNA, snoRNA, lncRNA, piRNA, tsRNA, srRNA, crRNA, tracrRNA, sgRNA, shRNA, ncRNA, mRNA, präkondensierte DNA, pDNA, ein Aptamer oder eine Kombination davon handelt.

7. Zusammensetzung nach einem der Ansprüche 1-6, wobei eine Zielzelle ausgewählt ist aus der Gruppe bestehend aus einem Neuron, einem Astrozyten oder einer Vorläuferzelle.

8. Zusammensetzung nach einem der Ansprüche 1-7, wobei die Zusammensetzung in Form von Nanopartikeln mit einem Durchmesser von etwa 15 nm bis etwa 300 nm, wie mit dynamischer Lichtstreuung (DLS) gemessen, vorliegt, wobei der Begriff "etwa" 10% plus oder minus des genannten Zahlenwerts bedeutet.

9. Zusammensetzung nach einem der Ansprüche 1-8, wobei die Zusammensetzung in Form von Nanopartikeln mit einem Durchmesser von etwa 80 nm bis 150 nm, wie mit dynamischer Lichtstreuung (DLS) gemessen, vorliegt, wobei der Begriff "etwa" 10% plus oder minus des genannten Zahlenwerts bedeutet.

10. Zusammensetzung nach einem der Ansprüche 1 bis 9, umfassend:

    (a) 40 MOL% 1,17-Bis(2-octylcyclopropyl)heptadecan-9-yl-4-(dimethylamino)butanoat oder ein pharmazeutisch unbedenkliches Salz davon;
    (b) 40 MOL% 1,2-Dioleoyl-sn-glycero-3-phospho-ethanolamin (DOPE);
    (c) 17,5 MOL% Cholesterin; und
    (d) 2,5 MOL% Polyoxyethylen(40)-stearat.

11. In-vitro-Verfahren zur Einführung einer in eine Zelle unter Beibehalten von Aktivität der Nukleinsäure und Lebensfähigkeit der Zelle, umfassend In-Kontakt-Bringen der Zelle mit der Zusammensetzung nach einem der Ansprüche 4-10.

12. In-vitro-Verfahren zur Modulation der Expression eines Zielpolynukleotids oder -polypeptids in einer Zelle unter Beibehalten der Zelllebensfähigkeit, umfassend In-Kontakt-Bringen der Zelle mit der Zusammensetzung nach einem der Ansprüche 4-10, wobei die Nukleinsäure zur Modulation der Expression eines Zielpolynukleotids oder -polypeptids fähig ist.

13. Verfahren nach Anspruch 11 oder Anspruch 12, wobei die Zelle ausgewählt ist aus einer Gruppe bestehend aus

einem Säuger-Neuron, einem Astrozyten oder einer Vorläuferzelle.

**14.** Verfahren zur Erzeugung eines Lipidnanopartikels, umfassend die Zusammensetzung nach einem der Ansprüche 1-10 mit der Fähigkeit zum Targeting von Neuronen, wobei das Verfahren Erhöhen des Verhältnisses von kationischem Lipid zu Strukturlipid im Lipidnanopartikel beinhaltet.

**15.** Verfahren zur Erzeugung eines Lipidnanopartikels, umfassend die Zusammensetzung nach einem der Ansprüche 1-10 mit der Fähigkeit zum Targeting von Astrozyten, wobei das Verfahren Vermindern des Verhältnisses von kationischem Lipid zu Strukturlipid im Lipidnanopartikel beinhaltet.

## Revendications

**1.** Composition à réactif de transfection comprenant :

(a) 40% en moles de 1,17-bis(2-octylcyclopropyl)heptadécan-9-yl-4-(diméthyl-amino)butanoate, ou un sel de celui-ci acceptable d'un point de vue pharmaceutique ;
(b) de la 1,2-dioléoyl-sn-glycéro-3-phospho-éthanolamine (DOPE) à 10-40% en moles ;
(c) du cholestérol à 10-20% en moles ; et
(d) un agent stabilisant à 2,5% en moles.

**2.** Composition de l'une quelconque de la revendication 1, dans laquelle l'agent stabilisant est choisi dans le groupe constitué par l'éther oléylique de polyoxyéthylène (20), l'éther laurylique de polyoxyéthylène (23), le stéarate de polyoxyéthylène (40) et un lipide conjugué au PEG.

**3.** Composition de la revendication 2, dans laquelle l'agent stabilisant est le 1,2-dimyristoyl-sn-glycérol, méthoxy-polyéthylène glycol (PEG-DMG).

**4.** Composition de l'une quelconque des revendications 1-3, comprenant en outre un acide nucléique.

**5.** Composition de la revendication 4, dans laquelle l'acide nucléique est un ADN, un ARN, un acide nucléique verrouillé, un analogue d'acide nucléique ou un plasmide capable d'exprimer un ARN.

**6.** Composition de la revendication 4, dans laquelle l'acide nucléique est un oligonucléotide antisens, un ribozyme, un ARNmi, un ARNr, un ARNt, un ARNsi, un ARNsa, un ARNsn, un ARNsno, un ARNlnc, un ARNpi, un ARNts, un ARNsr, un ARNcr, un ARNtracr, un ARNsg, un ARNsh, un ARNnc, un ARNm, un ADN précondensé, un ADNp, un aptamère ou une combinaison de ceux-ci.

**7.** Composition de l'une quelconque des revendications 1-6, dans laquelle une cellule cible est choisie dans le groupe constitué par un neurone, un astrocyte ou une cellule progénitrice.

**8.** Composition de l'une quelconque des revendications 1-7, la composition existant sous forme de nanoparticules ayant un diamètre allant de 15 nm environ à 300 nm environ, tel que mesuré par diffusion dynamique de la lumière (DLS), dans laquelle le terme "environ" signifie le nombre cité plus ou moins 10%.

**9.** Composition de l'une quelconque des revendications 1-8, la composition existant sous forme de nanoparticules ayant un diamètre allant de 80 nm à 150 nm environ, tel que mesuré par diffusion dynamique de la lumière (DLS), dans laquelle le terme "environ" signifie le nombre cité plus ou moins 10%.

**10.** Composition de l'une quelconque des revendications 1 à 9, comprenant :

(a) 40% en moles de 1,17-bis(2-octylcyclopropyl)heptadécan-9-yl-4-(diméthyl-amino)butanoate, ou un sel de celui-ci acceptable d'un point de vue pharmaceutique ;
(b) de la 1,2-dioléoyl-sn-glycéro-3-phospho-éthanolamine (DOPE) à 40% en moles ;
(c) du cholestérol à 17,5% en moles ; et
(d) du stéarate de polyoxyéthylène (40) à 2,5% en moles.

**11.** Procédé in vitro destiné à introduire un acide nucléique dans une cellule, tout en conservant l'activité de l'acide

nucléique et la viabilité de la cellule, comprenant une mise en contact de la cellule avec la composition de l'une quelconque des revendications 4-10.

**12.** Procédé in vitro destiné à moduler l'expression d'un polynucléotide ou d'un polypeptide cible dans une cellule, tout en conservant viabilité de la cellule, comprenant une mise en contact d'une cellule avec la composition de l'une quelconque des revendications 4-10, dans laquelle l'acide nucléique est capable de moduler l'expression d'un polynucléotide ou d'un polypeptide cible.

**13.** Procédé de la revendication 11 ou la revendication 12, dans lequel la cellule est choisie dans le groupe constitué par un neurone de mammifère, un astrocyte ou une cellule progénitrice.

**14.** Procédé de fabrication d'une nanoparticule lipidique, comprenant la composition de l'une quelconque des revendications 1-10, capable de cibler des neurones, le procédé incluant une augmentation du rapport du lipide cationique au lipide structurel dans la nanoparticule lipidique.

**15.** Procédé de fabrication d'une nanoparticule lipidique, comprenant la composition de l'une quelconque des revendications 1-10, capable de cibler des astrocytes, le procédé incluant une diminution du rapport du lipide cationique au lipide structurel dans la nanoparticule lipidique.

Fig. 1

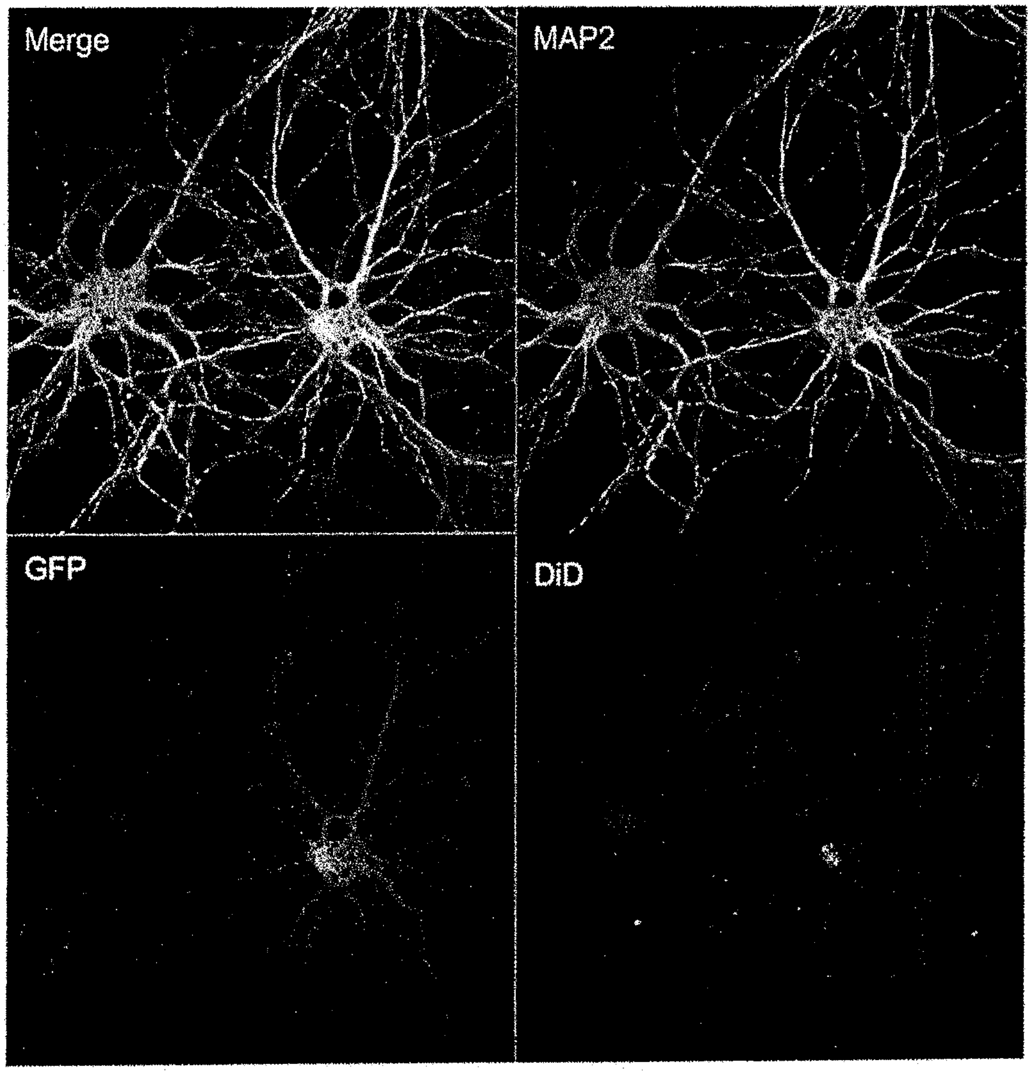

Fig. 2

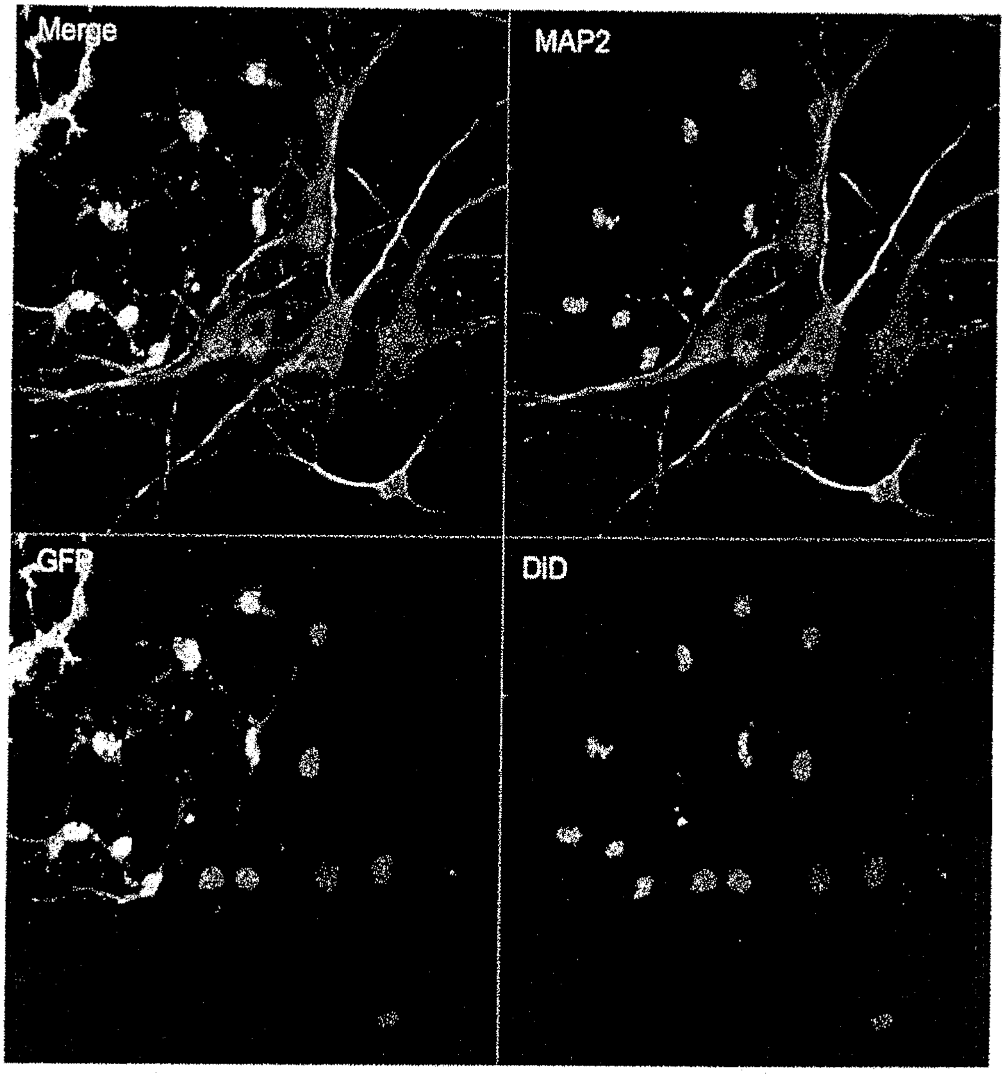

Fig. 3

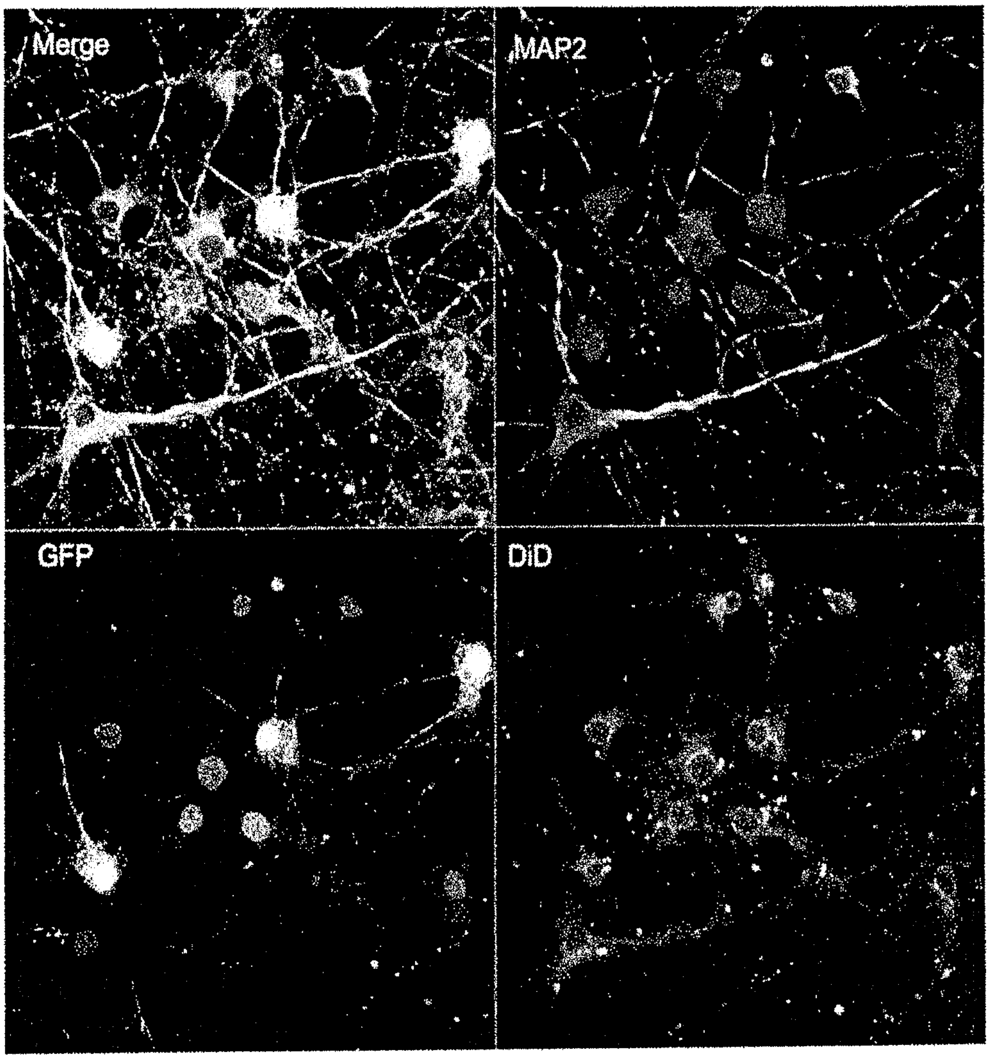

Fig. 4

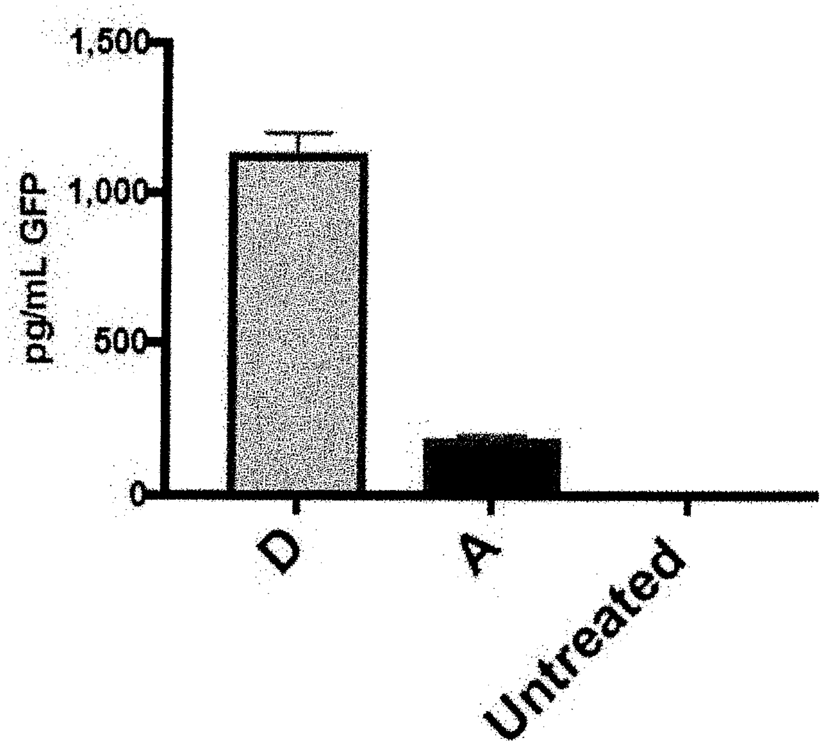

Fig. 5

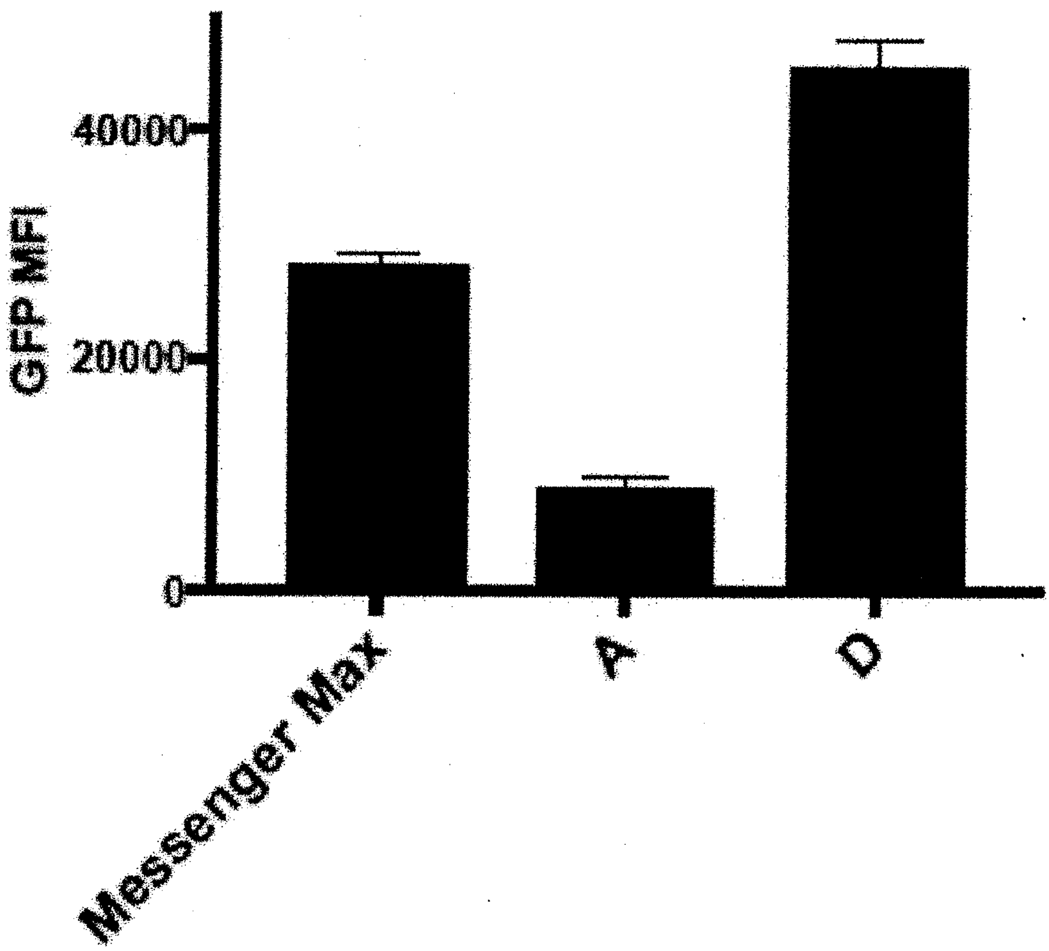

Fig. 6

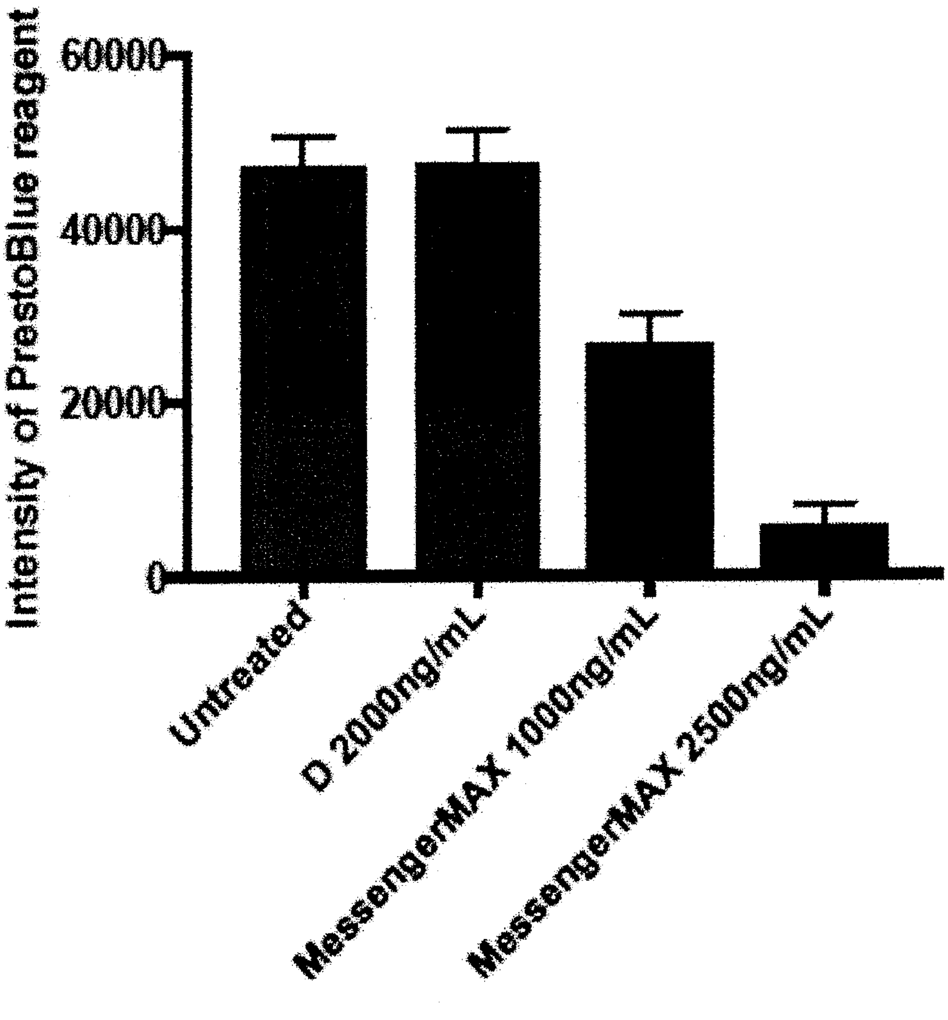
Intensity of PrestoBlue reagent
60000
40000
20000
0
Untreated
D 2000ng/mL
MessengerMAX 1000ng/mL
MessengerMAX 2500ng/mL

Fig. 7

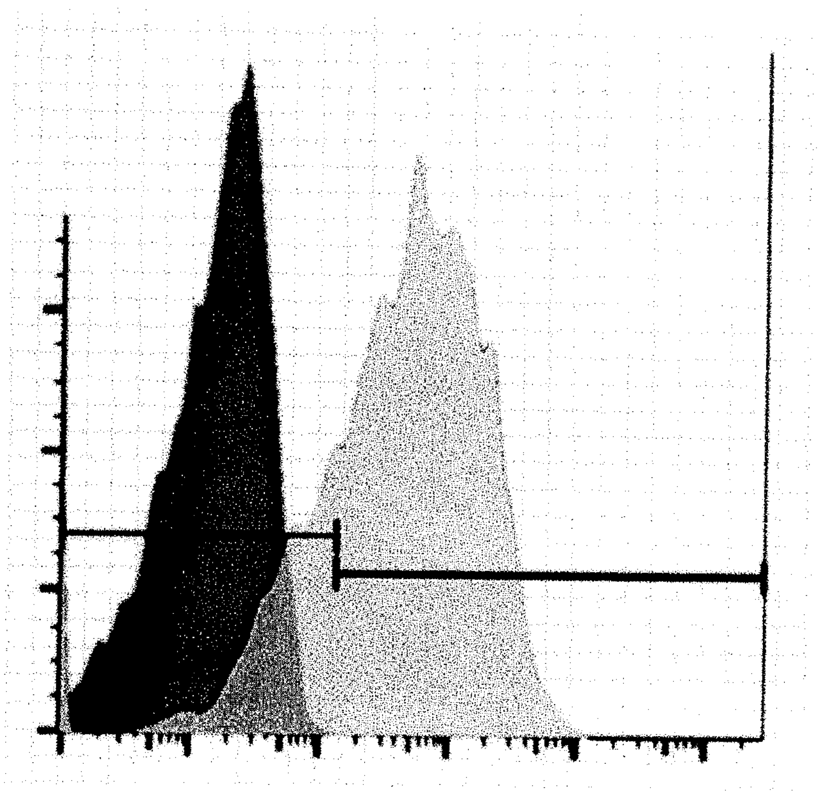

Fig. 8

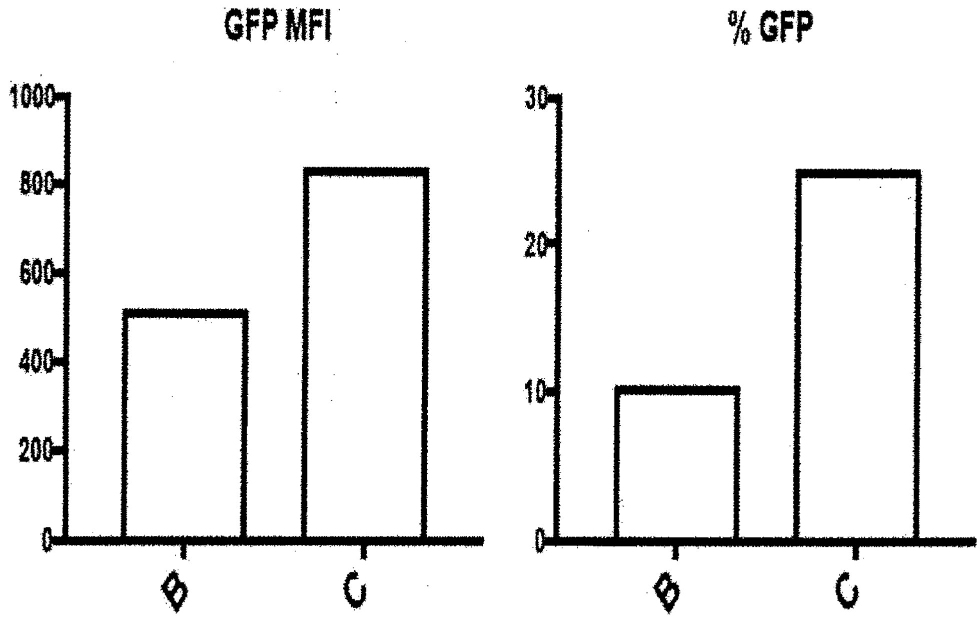

Fig 9

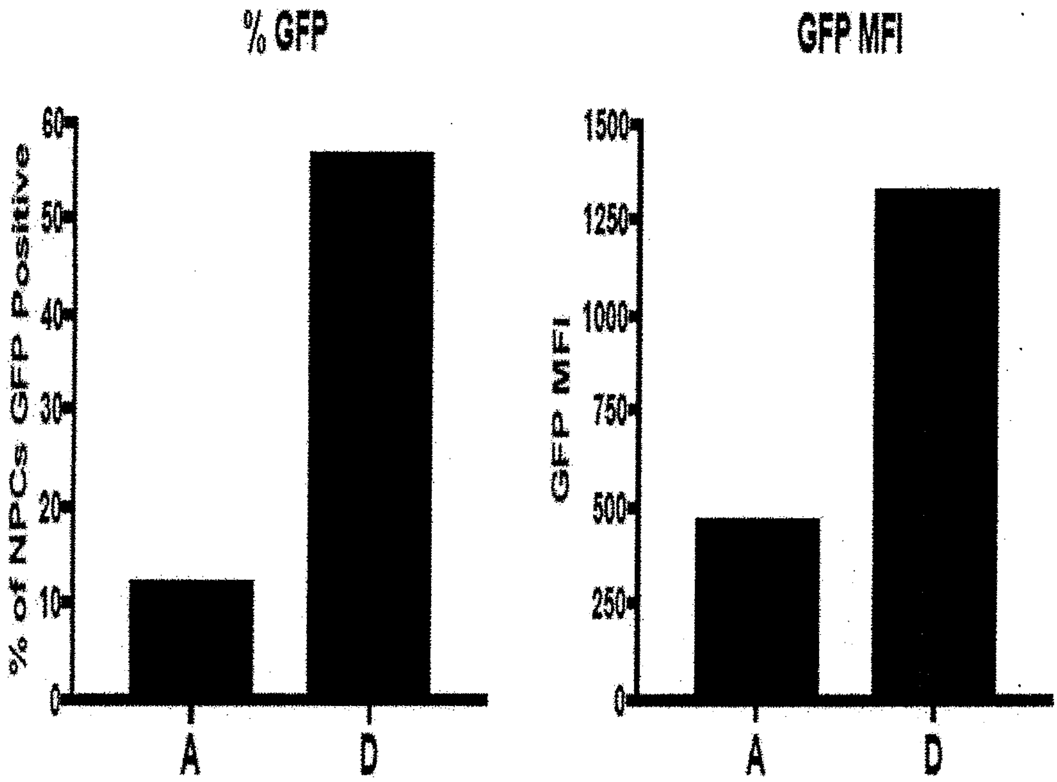
% GFP
% of NPCs GFP Positive
60
50
40
30
20
10
0
A
D
GFP MFI
GFP MFI
1500
1250
1000
750
500
250
0
A
D

Fig. 10

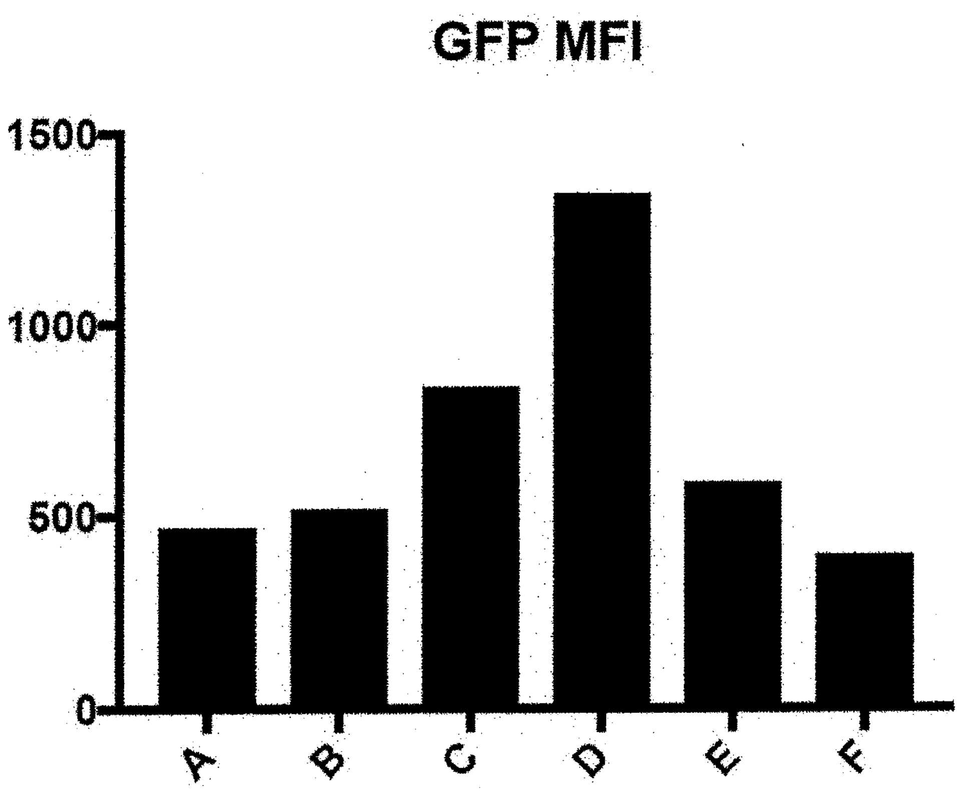

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 62403640 B
- WO 2014172045 A1
- WO 2009096558 A
- US 2013323269 A
- US 4835263 A, Nguyen
- WO 9822489 A
- WO 9839352 A
- WO 9914226 A
- US 6251666 B, Beigelman
- US 5753789 A, Chu
- EP 0540742 A, Shudo
- US 5432272 A, Eragen
- US 290672
- US 6433134 B
- US 5539082 A
- US 5527675 A
- US 5623049 A
- US 5714331 A
- US 5718262 A
- US 5736336 A
- US 5773571 A
- US 5766855 A
- US 5786461 A
- US 5837459 A
- US 5891625 A
- US 5972610 A
- US 5986053 A
- US 6107470 A
- WO 9604000 A
- US 9758795 B, Cullis
- US 20120276209
- US 20140328759, Cullis
- US 20160022580, Ramsay
- US 2016235688 A, Walsh
- WO 17117647 A
- WO 201711764 A, Wild
- US 20040262223
- WO 2017117647 A
- WO 62403640 A


### Non-patent literature cited in the description


- **AKHTAR, S. ; E. BASU ; S FAU - WICKSTROM ; R. L. WICKSTROM ; E FAU - JULIANO ; R. L. JULIANO.** *Interactions of antisense DNA oligonucleotide analogs with phospholipid membranes (liposomes),* 1991, 0305-1048
- **KAUFFMAN, K. J. ; M. J. WEBBER ; D. G. ANDERSON.** Materials for non-viral intracellular delivery of messenger RNA therapeutics. *J Control Release,* 2015
- **KAUFMANN K ; DORKIN ROBERT J ; YANG, JUNG H ; HEARTLEIN, MICHAEL W ; DE ROSA, FRANK ; MIR, FARYAL F ; FENTON, OWEN S ; ANDERSON, DANIEL G.** Optimization of Lipid Nanoparticle Formulations for mRNA Delivery in Vivo with Fractional Factorial and Definitive Screening Designs. *Nano Letters,* 2015, vol. 15, 7300-7306
- **LV, H. ; S. ZHANG ; B. WANG ; S. CUI ; J. YAN.** Toxicity of cationic lipids and cationic polymers in gene delivery. *Journal of Controlled Release,* 2006, vol. 114 (1), 100-109
- **MINGOZZI, F ; K. A. HIGH.** Immune responses to AAV vectors: overcoming barriers to successful gene therapy. *Blood,* 2013, vol. 122 (1), 23-36
- **O'MAHONY, A. M. ; B. M. GODINHO ; J. F. CRYAN ; C. M. O'DRISCOLL.** Non-viral nanosystems for gene and small interfering RNA delivery to the central nervous system: formulating the solution. *J Pharm Sci,* 2013, vol. 102 (10), 3469-3484
- **TAM, Y. Y. ; S. CHEN ; P. R. CULLIS.** Advances in Lipid Nanoparticles for siRNA Delivery. *Pharmaceutics,* 2013, vol. 5 (3), 498-507